# EUROPEAN PATENT SPECIFICATION

(11) **EP 4 182 001 B1**
(45) Date of publication and mention of the grant of the patent: **25.03.2026**
(21) Application number: 21749973.0
(22) Date of filing: 15.07.2021
(51) Int. Cl.: A61M 27/00, A61M 39/02, A61B 10/00

(54) **IMPLANTABLE CRANIAL MEDICAL DEVICE**
KRANIALE IMPLANTIERBARE MEDIZINISCHE VORRICHTUNG
DISPOSITIF CRÂNIEN MÉDICAL IMPLANTABLE

(30) Priority: 15.07.2020 US 202063052284 P; 11.06.2021 US 202163209835 P
(43) Date of publication of application: 24.05.2023
(73) Proprietor: Biogen MA Inc., Cambridge, MA 02142 (US)
(72) Inventor: ABRAMS, Daniel J., Aurora, Colorado 80010 (US); SCHAFER, Lisa Lynn, Aurora, Colorado 80010 (US); DATTA, Arindam, Hillsborough, Colorado 08844 (US); PRENTICE, Thomas R., Winter Haven, Florida 33884 (US); RUMPZA, Paul, New Brighton, Minnesota 55112 (US); TOTH, Felix Ernesto Robles, Duxbury, Massachusetts 02332 (US); JACOBS, Jordan, Randolph, Massachusetts 02368 (US); BUTZIGER, John, East Greenwich, Rhode Island 02818 (US)
(74) Representative: J A Kemp LLP
(86) International application number: PCT/US2021/041763
(87) International publication number: WO 2022/015941

(56) References cited:
- EP-A2- 0 995 460
- WO-A1-2018/023041
- US-A1- 2018 140 810

## Description

### FIELD

The present disclosure relates to, implantable cranial medical devices, such as devices for delivering fluid to cerebrospinal fluid (CSF), such as cerebral ventricles, withdrawing or draining fluid from CSF, such as cerebral ventricles, or delivering fluid to CSF and withdrawing or draining fluid from CSF. US 2018/140810 A1, WO 2018/023041 A1, and EP 0 995 460 A2 disclose background art to the invention.

### INTRODUCTION

Delivery of therapeutic agents to the central nervous system (CNS) and treatment of diseases of the CNS present challenges. For example, many therapeutic agents are not able to reach the brain at therapeutic concentrations when administered through traditional routes due to the blood-brain-barrier (BBB). In addition, systemic concentrations of therapeutic agents, or metabolites or degradation products thereof, may be undesirably high to achieve therapeutic levels in the brain when therapeutic agents that do cross the BBB are systemically administered.

Some devices and therapies have been developed to administer therapeutic agents to CSF to address some of these challenges. Such devices and therapies have typically been configured to deliver a bolus of therapeutic agent to a cerebral ventricle or to chronically administer the therapeutic agent to intrathecal space of the spinal canal. Such approaches have shortcomings for treatment of diseases of the brain. For example, such approaches lack the ability to achieve adequate spatially and temporally exposure of a therapeutic agent. Bolus administration of a therapeutic agent may not provide consistent therapeutically effective concentrations of the therapeutic agent, and intrathecal administration may not provide for sufficiently high concentrations of therapeutic agent in the brain due to, for example, gravitational forces and relatively limited CSF circulation.

Monitoring of a state a disease, subject or therapy, such as concentrations of therapeutic agents in the brain, may also present challenges. For example, accurate estimates of concentrations of therapeutic agents in the brain may not always be readily achieved through routine blood or urine analysis. In addition, withdrawing CSF to obtain more accurate estimates of central therapeutic agent concentrations is invasive and may present risks.

For those therapies that include direct intracerebral ventricular delivery of therapeutic agents through, for example, an implantable infusion pump or port, CSF may be withdrawn through a catheter configured to deliver the therapeutic agent to the CSF from the pump or port. However, withdrawing CSF through the same lumen of a catheter that has been used to deliver therapeutic agent may not provide a clean sample of CSF. That is, the CSF may include therapeutic agent that was present in the lumen of the catheter and thus may not provide for accurate estimates of concentration of the therapeutic agent in the CSF. In addition, the CSF or the components of the CSF that maybe measured may interact with the material defining the lumen of the catheter, which may adversely affect subsequent delivery of the therapeutic agent. Furthermore, withdrawing CSF through the same lumen of the catheter that has been used to delivery therapeutic agent requires an interruption in the delivery of the therapeutic agent. The interruption of delivery can lead to cessation of therapy and other substantial consequences.

The use of separate catheters or separate lumens of a catheter for delivering therapeutic agents to the cerebral ventricles and for withdrawing CSF from the cerebral ventricles has been proposed. However, designing and manufacturing devices, such as access ports, that may couple to two or more catheters or a catheter having more than one lumen for delivering fluid to or from cerebral ventricles presents challenges, particularly if the devices are configured to be implanted under the scalp or skin of a subject. For example, a subject, such as a human patient, may tolerate a limited height of a device, such as a port that is coupled to the catheter or catheters, above the skull when the device is implanted under the scalp. In addition, devices implanted under the scalp or skin should have an external shape and profile configured to minimize erosion of the skin and patient discomfort and facilitate safe and efficient surgical implantation. An additional challenge relates to ensuring a secure connection between catheters and the devices to which they are connected. For example, it may be difficult to couple a catheter to an access port after the port is positioned in a subject. Further challenges relate to reproducible access to the implanted port. For example, proper positioning of the port should be maintained to ensure repeated access through the skin of a subject over extended periods of time. Such challenges result from the limited space available for such devices to be implanted, multifunctionality of the devices, and the need for secure connection between a catheter and the access port.

At least in part because of these challenges, a device of suitable size, shape, and functionality for connecting to more than one catheter or a catheter with more than one lumen for delivering fluid to or from a CSF space, such as a cerebral ventricle, of a subject has not previously been developed.

### SUMMARY

The present invention relates to an implantable cranial device that comprises two or more separate fluid flow paths. The implantable cranial device may be coupled to multiple single lumen catheters, to one or more multi-lumen catheters, or to one or more single lumen catheters and to one or more multi-lumen catheters, with each lumen being independently in fluid communication with one of the separate fluid flow paths. One or more of the separate fluid flow paths may be used, for example, to deliver therapeutic agents to a cerebral ventricle through one or more lumens of one or more catheters. One or more of the separate fluid flow paths may be used, for example, to withdraw CSF from a cerebral ventricle through one or more lumens of the one or more catheters.

The implantable cranial device is able to accommodate multiple separate fluid flow paths, in part, because a portion of the device is configured to be implanted within a burr hole in a skull and a portion is configured to be implanted between the skull and the scalp. The added space afforded by the portion implanted within the burr hole permits the implantable cranial device to include multiple separate fluid flow paths, while meeting design considerations to allow for safe and efficient implantation under the scalp.

According to an aspect of the present invention, an implantable cranial medical device comprises a first fluid flow path, a second fluid flow path, an upper flange portion, and a lower portion. The upper flange portion is configured to rest on a skull of a subject about a burr hole. The lower portion is configured to be placed within the burr hole. The first fluid flow path extends from a first opening in the upper flange portion to a first opening in the lower portion. The second fluid flow path extends from a second opening in the upper flange portion to a second opening in the lower portion.

According to another aspect of the present disclosure, an implantable cranial medical device comprises a first fluid flow path, a second fluid flow path, an upper flange portion, and a lower flange portion. The upper flange portion has a width in a range from 15 millimeters to 30 millimeters. The lower portion has a width in a range from 10 millimeters to 20 millimeters. The upper flange portion has a greater width than the lower portion. The first fluid flow path extends from a first opening in the upper flange portion to a first opening in the lower portion. The second fluid flow path extends from a second opening in the upper flange portion to a second opening in the lower portion.

The implantable cranial devices may include, or may be configured to be coupled to, for example, multiple single lumen catheters or to a multi-lumen catheter at, for example, the bottom of the lower portion such that one catheter lumen is in communication with the first fluid flow path and another catheter lumen is in communication with the second fluid flow path. Preferably, the catheter or catheters are coupled to the implantable cranial medical device prior to implanting in a subject to avoid problems with securely connecting the catheter or catheters to the implantable cranial medical device after the distal ends of the catheters have been positioned in the subject. More preferably, the implantable cranial medical device includes the catheter or catheters, or the catheter or catheters are coupled to the implantable cranial medical device by a manufacturer, to ensure secure connection of the catheter or catheters. Preferably, the catheter is a multi-lumen catheter because placement and implantation of multiple catheters may add surgical complexity.

At least one lumen of at least one catheter may be placed in fluid communication with a subject's CSF such that fluid may be delivered to or withdrawn from the subject's CSF through the first or second flow path of the implantable cranial medical device. In some embodiments, first and second lumens of one or more catheters are placed in fluid communication with a cerebral ventricle of a subject such that fluid may be delivered to or withdrawn from the cerebral ventricle through the first and second flow paths of the implantable cranial medical device. The catheter or catheters are preferably configured such that fluid may be delivered to or withdrawn from the cerebral ventricle through one of the first and second flow paths of the implantable cranial medical device without interference or mixing of the fluids that are being delivered to, or withdrawn from, the cerebral ventricle through the other of the first and second flow paths. In some embodiments, the catheters or catheters are configured such that one lumen may deliver fluid to brain tissue other than a cerebral ventricle and another lumen may deliver fluid to or withdraw fluid from a cerebral ventricle.

Also described herein are methods of implanting the implantable cranial medical devices and associated catheter or catheters, as well as methods of treating, monitoring, or treating and monitoring a disease of state of a subject using the implantable cranial medical devices. The described methods do not form part of the present invention.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF THE DRAWINGS

**FIG. 1** is a schematic side view of an embodiment of an implantable cranial medical device.
**FIG. 2** is a schematic bottom view of an embodiment of the implantable cranial medical device depicted in **FIG. 1****.**
**FIG. 3** is a schematic top view of an embodiment of the implantable cranial medical device depicted in **FIGS. 1** and **2****.**
**FIG. 4** is a schematic side view of an embodiment of an implantable cranial medical device.
**FIG. 5** is a schematic top view of an embodiment of the implantable cranial medical device depicted in **FIG. 4****.**
**FIG. 6** is a schematic side view of the implantable cranial medical device depicted in **FIG. 4** in which the housing is sectioned.
**FIG. 7** is a schematic perspective view showing a bottom of an embodiment of the implantable cranial medical device depicted in **FIGS. 4-6****.**
**FIG. 8** is a schematic cross-sectional view of an embodiment of a dual lumen brain catheter.
**FIG. 9** is a schematic longitudinal sectional view of a distal portion of a brain catheter.
**FIG. 10** is a schematic longitudinal sectional view of a distal portion of a brain catheter.
**FIG. 11** is an exploded perspective view of an embodiment of an implantable cranial medical device showing components of a port and an external catheter connector.
**FIG. 12** is a photograph of a cut away of a housing of an embodiment of an implantable cranial medical device.
**FIG. 13** is a perspective view of an embodiment of an implantable cranial medical device coupled to a brain catheter and a stylet employed for implanting the brain catheter while the brain catheter is coupled to the implantable cranial medical device.
**FIG. 14** is a schematic sectional view illustrating an embodiment of an implantable cranial medical device, brain catheter, and external catheter implanted in a patient.
**FIG. 15** is a schematic sectional view illustrating some components of an embodiment of implantable cranial device with fluid flow pathways shown in dashed lines.
**FIG. 16** is a schematic drawing of a system including an implantable cranial medical device and an external device.
**FIG. 17** is a schematic drawing of a system including an implantable cranial medical device and two external devices.
**FIGS. 18-21** are schematic illustrations of some components of embodiments of systems described herein.
**FIG. 22** is a schematic sectional view of embodiments of a port that may be connected to an implantable cranial medical device.
**FIG. 23** is a schematic drawing showing infusion of agents into a cerebral ventricle and aspiration of cerebral spinal fluid from the ventricle using a catheter according to an embodiment of the invention.
**FIG. 24** is an image of an embodiment of an implantable cranial device implanted in a skull of a patient.

While the invention is susceptible to various modifications and alternative forms, specific embodiments thereof are shown by way of example in the drawings and may herein be described in detail. The drawings may not be to scale.

Like numbers used in the figures refer to like components and steps. However, it will be understood that the use of a number to refer to a component in a given figure is not intended to limit the component in another figure labeled with the same number. In addition, the use of different numbers to refer to components in different figures is not intended to indicate that the different numbered components cannot be the same or similar to other numbered components

### DETAILED DESCRIPTION

The present invention relates to an implantable cranial medical device. The implantable cranial medical device comprises first and second fluid flow paths. The fluid flow paths are separate and isolated. The implantable cranial medical device may comprise one or more catheters, or may be configured to operably couple to one or more catheters, for delivering fluid to, or withdrawing fluid from, a tissue or fluid of a central nervous system of a subject. Preferably, the implantable cranial medical device includes a multi-lumen catheter or is configured to operably couple to a multi-lumen catheter, where a first lumen of the multi-lumen catheter is in fluid communication with the first fluid flow path and a second lumen of the multi-lumen catheter is in fluid communication with the second fluid flow path. The first fluid flow path and the first lumen may be configured to deliver fluid to, or withdraw fluid from, a first target brain location. The second fluid flow path and the second lumen may be configured to deliver fluid to, or withdraw fluid from, a second target brain location. The first and second target brain locations may be the same or different.

Preferably, at least one of: (i) the first fluid flow path and first lumen; and (ii) the second fluid flow path and second lumen, is configured to withdraw CSF from a subject, for example, from a cerebral ventricle. For purposes of the present disclosure, withdrawing CSF from a CSF-containing space, such as a cerebral ventricle, includes aspirating or draining CSF from the CSF-containing space. In some embodiments, both (i) the first fluid flow path and first lumen and (ii) the second fluid flow path and second lumen, are configured to deliver fluid to, or withdraw fluid from, a CSF-containing space of a subject. Preferably, one fluid flow path and lumen are used to deliver fluid to a CSF-containing space, such as a cerebral ventricle, and the other fluid flow path and lumen are used to withdraw fluid from a CSF-containing space, such as a cerebral ventricle.

The implantable cranial device is able to accommodate multiple separate fluid flow paths, in part, because a portion of the device is configured to be implanted within a burr hole in a skull and a portion is configured to be implanted between the skull and the scalp. The added space afforded by the portion implanted within the burr hole permits the implantable cranial device to include multiple separate fluid flow paths, while meeting design considerations to allow for safe and efficient implantation under the scalp.

The implantable cranial medical device comprises an upper flange portion and a lower portion. The lower portion may be sized and shaped to be disposed within a burr hole in a skull of a subject. Accordingly, the size and shape of the lower portion may vary depending on the size and shape of the burr hole, or the size of the burr hole may be determined by the size and shape of the lower portion. Preferably, the clearance between an exterior side surface of the lower portion and the skull is small when the lower portion is positioned in the burr hole. The smaller the clearance between the skull and the exterior side surface of the lower portion, the larger the volume of the lower portion. Larger volumes of the lower portion may facilitate incorporation of both the first and second flow paths within the device. Smaller clearances between the exterior side surface of the lower portion and the burr hole also improve stability of the implantable cranial device relative to the skull. Improved stability may be manifest in less movement of the implantable cranial device relative to the skull.

In some embodiments, the clearance between the exterior side surface of the lower portion and the skull, when the lower portion is disposed in the burr hole, is between 0 mm and 5 mm. For example, the clearance between the exterior side surface of the lower portion and the skull, when the lower portion is disposed in the burr hole, may be between 0.1 mm and 3 mm, such as between 0.2 mm and 2 mm.

In some embodiments, the lower portion has a width in a range from about 10 millimeters to about 20 millimeters. For example, the lower portion of the housing may have a width in a range from about 10 millimeters to about 14 millimeters, such as from about 11 millimeters to about 13 millimeters. A burr hole typically is cylindrical. In some embodiments, the burr hole may have a diameter of about 14 millimeters. The lower portion of the device may have a generally circular cross-section, and the width of the lower portion may be a diameter.

When the implantable cranial medical device is implanted, a bottom major surface of the lower portion preferably does not extend substantially beyond the bottom of the burr hole. Accordingly, the height of the lower portion of the housing of the implantable cranial medical device may vary depending on the thickness of the skull of the subject into which the device is implanted. As an example, a thickness of a human adult skull may typically be in a range from about 6.5 millimeters to about 7 millimeters.

In some embodiments, the height of the lower portion of the implantable cranial medical device is in a range from about 3 millimeters to about 7 millimeters. For example, the height of the lower portion may be in a range from about 4 millimeters to about 6 millimeters or from about 4.5 millimeters to about 5.5 millimeters.

The upper flange portion is preferably configured to rest on a skull of a subject above the burr hole. For purposes of this disclosure, the upper flange portion will be considered to rest on the skull if one or more intervening structures are placed between the bottom of the upper flange portion and the skull. The upper flange portion preferably has a width greater than the burr hole so that the upper flange portion may rest on the skull above the burr hole when the lower portion is positioned in the burr hole. Accordingly, the upper flange portion preferably has a width greater than the lower portion.

The upper flange portion may have a top surface and a bottom surface. The bottom surface of the upper flange portion may laterally extend relative to the lower portion. The bottom surface of the upper flange portion may be annular. The bottom surface of the upper flange portion may be generally flat. For purposes of the present disclosure, "generally flat," in the context of the bottom surface of the upper flange portion includes slightly curved to approximate curvature of a skull. The bottom surface may be generally flat and annular.

The upper flange portion may have any suitable width. In some embodiments, the width is defined by the bottom surface of the upper flange portion. In some embodiments, the upper flange portion has a width in a range from about 15 millimeters to about 30 millimeters. For example, the upper flange portion may have a width in a range from about 18 millimeters to about 28 millimeters, or from about 22 millimeters to about 26 millimeters. The upper flange portion may have an outer surface that has a generally circular cross section. Accordingly, the width of the upper flange portion may be a diameter.

The upper flange portion may have any suitable height. Preferably, the height of the upper flange portion is sufficiently small to be well tolerated by a subject when the implantable cranial medical device is implanted under a scalp of the subject. The height of the upper flange portion may define the distance that the implantable cranial medical device extends above the skull when implanted in the subject.

In some embodiments, the height of the upper flange portion of the implantable cranial medical device is in a range from about 3 millimeters to about 8 millimeters. For example, the height of the upper flange portion may be in a range from about 4 millimeters to about 6.5 millimeters or from about 5 millimeters to about 6.5 millimeters.

The implantable cranial medical device may have any suitable overall height. In some embodiments, the height of the device from the bottom of the lower portion to the top of the upper flange portion of the housing is in a range from about 6 millimeters to about 15 millimeters, such as from about 8 millimeters to about 12 millimeters.

The top surface of the upper flange portion may have any suitable shape. Preferably, the top surface or the transition from a side surface to the top surface has no sharp edges. In some embodiments, the top surface of the housing has a generally convex shape.

In some embodiments, the bottom surface of the upper flange portion of the housing is generally flat and annular and the top surface is generally convex.

In some embodiments, the thickness or height of the upper flange portion increases moving from an outer edge of the upper flange portion towards the center the upper flange portion.

The implantable cranial medical device comprises a housing. The housing defines an exterior surface of the device. The housing defines an external surface of the upper flange portion and an external surface of the lower flange portion.

The housing may be formed from one or more parts. In some embodiments, the housing comprises a single part that defines the exterior surface the upper flange portion and the lower portion of the housing. The housing may be formed from more than one part, the parts may be connected in any suitable manner. As an example, different parts of the housing may be secured relative to one another by threaded engagement, snap fit engagement, interference fit engagement, may be welded, adhered, or otherwise bonded to one another, or the like, or combinations thereof. Preferably, different parts of the housing are connected to one another in a fluid tight manner. Preferably, different parts of the housing are welded together.

The housing of the implantable cranial medical device may comprise any suitable material. For example, the housing of the implantable cranial medical device may be formed from one or more of a metallic material, a plastic material, a ceramic material, and a glass material. For example, the housing may comprise one or more of a high performance thermoplastic or relatively rigid plastic material, such as polyurethane, polycarbonate, polysulfone, polyether ether ketone (PEEK), nylon, and Ultra High Molecular Weight Polyethylene (UHMWPE); and a biocompatible metal, such as a stainless steel alloy, titanium, and nitinol. Preferably, the material is compatible with magnetic resonance imaging (MRI). Preferably, the housing comprises a biocompatible material or comprises an exterior biocompatible coating.

In some embodiments, implantable cranial medical device comprises a sleeve configured to cover one or more portions of the housing. The sleeve may be formed from one or more materials that are softer, more compliant, or softer and more compliant than the housing. The sleeve may be formed from any suitable material or materials. For example, the sleeve may comprise one or more of silicone and a thermoplastic elastomer. The sleeve is preferably biocompatible. The sleeve may be coated with or comprise a lubricious material, such as a hydrogel. The sleeve may serve to absorb impact that may occur to the patient's skin over the implantable cranial medical device, which may reduce potential damage to the device or the patient. The sleeve may serve to reduce tissue erosion over time, particularly if the sleeve is formed from lubricious material or a material that is softer or more compliant than the housing. The sleeve may comprise one or more antimicrobial agent, such as antimicrobial silver and antibiotics. For example, the sleeve may be impregnated with one or antibiotics, such as minocycline and rifampin.

The first and second fluid flow paths of the implantable cranial medical device may be configured in any suitable manner. The first and second fluid flow paths each extend within an interior of the housing, from the upper flange portion of the device to the lower portion of the device. The first fluid flow path extends from a first opening in the upper flange portion to a first opening in the lower portion, and the second fluid flow path extends through the housing from a second opening in the upper flange portion to a second opening in the lower portion.

The fluid flow paths may be formed in any suitable manner and form any suitable material or materials. The fluid flow paths, or portions thereof, may comprise passageways formed from one or more parts. In some examples, the passageway is formed from a lumen within tubing. In some examples, the passageway is formed from a channel in a solid part that is coupled to a second part to enclose the passageway. In some embodiments, the fluid flow paths are formed by the housing. For example, the fluid flow paths may be formed in a mold of the housing, micromachined in a component of the housing, may be formed by void spaced generated by assembling components of the housing, or the like. Preferably, the material lining the passageway is compatible with fluid that flows through the passageway, such as a fluid containing a therapeutic agent or CSF. Preferably, the therapeutic agent or excipients or CSF do not sorb to or react with the material defining the passageway. For purposes of the present disclosure, "sorb" refers to one or both of adsorb and absorb.

The structural material forming the passageway may be compatible with the fluid or the structural material may be coated with a compatible material.

Any suitable material or material may define the fluid flow paths of the implantable cranial medical device. In some embodiments, the material defining the fluid flow paths comprises one or more of metallic material, polymeric material, ceramic material, or glass material. For example, the material defining the fluid flow paths may comprise one or more of a high performance thermoplastic or relatively rigid plastic material, such as polyurethane, polycarbonate, polysulfone, polyether ether ketone (PEEK), nylon, and Ultra High Molecular Weight Polyethylene (UHMWPE); and a biocompatible metal, such as a stainless steel alloy, titanium, and nitinol.

The implantable cranial medical device may comprise one or more filter positioned and arranged to filter fluid flowing through the first fluid flow path, the second fluid flow path, or the first and second fluid flow paths. Preferably the filter has a pore size of 0.45 microns or less, such as 0.22 microns or less, or 0.2 microns or less. Filters having pore sizes of 0.45 microns or less may effectively filter microbes, such as bacteria. Filters having pore sizes of 0.22 microns or less mat effectively filter viruses.

Preferably, a fluid flow path that is configured for withdrawing (aspirating) CSF does not comprise a microbial filter. The ability to withdraw CSF through a microbial filter over time may diminish if a microbial filter is present in a passageway through which the CSF is withdrawn. The ability to withdraw CSF through a microbial filter over time may be due to accumulation of cells and CSF proteins on the filter over time, which may clog the filter. Diminished ability to withdraw CSF through the first fluid pathway of the device may reduce usability of the device (e.g., only a fluid delivery pathway may remain useable). In addition, if a microbial infection is present in the CSF, the microbe responsible for the infection may be removed from the CSF if it were passed through a microbial filter. Thus, the presence of a microbial filter in a fluid flow path configured to withdraw CSF may prevent the ability to detect an infection in withdrawn CSF. For at least these reasons, a fluid flow path configured to withdraw CSF preferably does not include a microbial filter.

In some embodiments, the second fluid flow path comprises a microbial filter and the first fluid flow path does not include a microbial filter. The first fluid flow path may be configured for withdrawal of CSF. The second fluid flow path may be configured for introduction of therapeutic fluid.

The implantable cranial medical device may comprise any suitable connector or port to permit fluid to flow to or from a flow path of the device through a separate device, such as a catheter, needle, or the like. The connector or port may be formed, at least in part, the upper flange portion or the lower portion of the device. For example, the first or second openings of the upper flange portion or the lower portion may define the connector or port, or a portion thereof. The connector or port may be, at least in part, separate from, but operably coupled to, the upper flange portion or the lower portion. For example, the connector or port may be operably coupled to the first or second openings of the upper flange portion or the lower portion to place the connector or port in communication with the fluid flow path.

The implantable cranial medical device may comprise one or more brain catheter connectors. For purposes of the present disclosure, a "brain catheter connector" is a connector operably couplable to a catheter that is configured to extend from the implantable cranial medical device into a brain of a subject, such as into a CSF-containing space (e.g., a cerebral ventricle), when implanted in the subject.

A brain catheter connector of the implantable cranial medical device may be coupled to a brain catheter in any suitable manner. For example, the brain catheter and the brain catheter connector may comprise quick-release couplings, luer lock fittings, snap connect couplings, or the like. In some examples, the brain catheter is configured to be coupled to the brain catheter connector via interference fit. The brain catheter connector may comprise a fitting, which may comprise external barbs, configured to be inserted into a lumen of the brain catheter to retain the brain catheter relative to the brain catheter connector and to place the lumen of the brain catheter in fluid communication with the fluid flow path of the implantable cranial medical device.

In some embodiments, the brain catheter connector comprises a first portion of a compression fitting and a second portion of the compression fitting is placed over the catheter. Connecting the first and second portion of the compression fitting may compress the external catheter to secure the catheter to the external catheter connector. For example, the compression fitting may cause the catheter to compress against external barbs. The second portion of the compression fitting may comprise a compression ferrule and a connection element to connect to the first portion of the fitting on the external catheter connector.

A brain catheter connector may be positioned at any suitable location of the implantable cranial medical device. Preferably, the brain catheter connector is located at a bottom of the device, such as at a bottom of the lower portion of the housing. For example, the brain catheter connector may extend from a bottom major surface of the lower portion of the housing. While the bottom major surface of the lower portion preferably does not extend beyond the bottom of a burr hole in which is placed, the brain catheter connector may extend beyond the bottom of the burr hole.

Preferably, the brain catheter connector comprises a first lumen in communication with the first fluid path of the device and comprises a second lumen in communication with the second fluid path of the device. Preferably, such a catheter connector is configured to connect with a dual lumen brain catheter. Upon coupling the dual lumen brain catheter to the brain catheter connector, a first lumen of the brain catheter may be placed in fluid communication with the first lumen of the brain catheter connector, and a second lumen of the brain catheter may be placed in fluid communication with the second lumen of the brain catheter connector. The lumens of the brain catheter may be used to carry fluid to or from one or more regions of the central nervous system. Preferably, at least one lumen of the brain catheter is in placed in fluid communication with a CSF-containing space. CSF exits the foramen of Magendie and Luschka to flow around the brainstem and cerebellum. CSF flows within the subarachnoid space. CSF is produced in the ventricular system of the brain and communicates freely with the subarachnoid space via the foramen of Magendie and Luschka. The lumen of the brain catheter may be placed in communication with CSF anywhere that the CSF is accessible. For example, the lumen may be placed in communication with an intrathecal space, a cisterna magna, a subarachnoid space or a cerebral ventricle. Preferably, the lumen is placed in communication with a cerebral ventricle. Preferably, the cerebral ventricle is a lateral cerebral ventricle.

Preferably, the brain catheter is operably coupled to the brain catheter connector before the brain catheter is implanted. In some embodiments, the brain catheter is connected to the brain catheter connector by a manufacturer. A brain catheter may be permanently or reversibly coupled to the brain catheter connector.

A brain catheter may have any suitable length. Preferably, the brain catheter has a length sufficient to extend to a CSF-containing space, such as a cerebral ventricle, when coupled to the cranial medical device when implanted. In some embodiments, the brain catheter has a length from about 55 millimeters to about 80 millimeters, such as from about 60 millimeters to about 70 millimeters, or from about 62 millimeters to about 68 millimeters. Such lengths may be suitable for the brain catheter to extend to a lateral cerebral ventricle.

The brain catheter may have any suitable outer diameter. Preferably, the outer diameter is from about 2 mm to about 4 mm, such as from about 2 mm to about 3 mm, or from about 2 mm to about 2.5 mm.

Preferably, the brain catheter comprises two lumens. The two lumens may be oriented in any suitable manner. For example, the two lumens may be concentric, such as a lumen within a lumen (or catheter within a catheter) or may be side-by-side. Preferably, the outer surface of the brain catheter has a substantially circular cross-sectional shape.

The brain catheter may comprise an inner wall running along the length of the catheter. The inner wall may separate the two lumens of the brain catheter. The brain catheter may comprise two semicircular (or D-shaped) cross-sectional shaped lumens running along the length of the catheter. The semicircular lumens may be of any suitable size. For example, the inner dimension of the semicircular lumens at their largest width may be in a range from about 0.9 millimeters to about 1.5 millimeters, such as from about 1.1 millimeters to about 1.5 millimeters, or from about 1.2 millimeters to about 1.4 millimeters. The inner dimension of the semicircular lumens at their smallest width may be in a range from about 0.4 millimeters to about 0.7 millimeters, such as from about 0.5 millimeters to about .6 millimeters, or from about 0.55 millimeters to about 0.6 millimeters. The inner dimensions of the first and second lumens may be the same or different. Preferably, the inner diameter of the first and second lumen are the same or substantially the same (e.g., do not vary by more than 10%).

The brain catheter may comprise any suitable material. The material is preferably biocompatible and compatible with a therapeutic fluid that may be delivered through a lumen of the catheter. The material is preferably biocompatible and compatible with CSF that may be flow through a lumen of the catheter. Preferably, the material is biodurable. A biodurable material is a material that is compositionally and structurally stable for extended periods of time in a biological environment. Products made from such materials should not exhibit substantial breakdown, degradation, erosion, or deterioration of mechanical properties relevant to their employment when exposed to biological environments for periods of time commensurate with the use of the implantable device. An intended biological environment can be understood to be *in vivo, i.e.* associated with an implantable device in a patient. The period of implantation of a brain catheter described herein may be weeks, months, or years. For example, a brain catheter may be made from materials that are biodurable for at least 29 days, such as at least one year, at least three years, or at least five years.

Any suitable homopolymer, copolymer, blends of polymers or combinations of polymers may be used to form the brain catheter. Preferably, the polymers used to make the catheters are flexible during both fabrication and assembly. Preferably, the catheters are formed from materials that result in a flexible and soft catheter during its *in vivo* implantation period.

Preferably, the chemical composition and molecular structure is selected so that the catheter materials are flexible and soft but still remain biodurable and resist substantial breakdown, degradation, erosion, or deterioration of mechanical properties when exposed to therapeutic agents or excipients delivered over extended periods of time. Flexibility and softness are characteristics that tend to cause a catheter to lack biodurability and long-term compatibility in oxidative, hydrolytic, and body fluid contact environments. Accordingly, the choice of polymers should be carefully selected to achieve sufficient compatibility with the therapeutic agent and excipients, biodurability, flexibility, and softness.

Depending on compatibility with the therapeutic agent and excipients to be employed, the brain catheter may comprise cross-linked silicone, which may form flexible catheters. The glass transition temperature of cross-linked silicone may depend on the cross-link density. In some embodiments, the brain catheter may comprise cross-linked silicone having a Tg below - 40°C, such as below -90°C. In some instances, components of therapeutic fluids may interact with a cross-linked silicone catheter. In some instances, components of therapeutic fluids may leach or extract components or parts of a cross-linked silicone catheter. Swelling and leaching or extraction may affect the biodurable nature of soft and flexible cross-linked silicone and its capability to maintain dimensions or wall thickness and structure to provide multiple stable fluid paths over extended periods of time.

In some embodiments, a brain catheter comprises polyurethane. Preferably, the brain catheter comprising polyurethane resists degradation in oxidative, hydrolytic, and body fluid contact environments. The polyurethane may comprise biostable hard and soft segments. The polyurethane may comprise a higher hard segment content. Such polyurethanes may resist degradation in oxidative, hydrolytic, and enzymatic changes under physiological conditions and body fluid contact. In some embodiments, the brain catheter comprises a polyurethane containing hard and soft segments that are formed by reacting a diol or polyol (an alcohol with more than two reactive hydroxyl groups per molecule) with a diisocyanate or a polymeric isocyanate in the presence of suitable catalysts and additives. The isocyanates (sometime chain extended with diols) form the hard segments and the polyols form the soft segments, with the segments linked by the urethane bonds formed from the reaction between the polyols and diisocyanate.

In some embodiments, the hard segment of the polyurethane comprises of an aromatic isocyanate. In some embodiment, the hard segment of the polyurethane comprises an aliphatic isocyanate. Preferably, the hard segment comprises an aromatic isocyanate as.

In some embodiments, the soft segment of the polyurethane comprises of a polyether. In some embodiments, the soft segment of the polyurethane comprises of a polycarbonate. The soft segment may provide suitable flexibility for use in single lumen catheters and multi-lumen catheters.

In some embodiments, a brain catheter comprises a polyurethane that is semi-crystalline with higher hardness. The semi-crystalline nature of the polyurethane polymers makes them resistant to swelling and leaching when in contact with CSF, therapeutic fluids, and therapeutic fluids having high concentration therapeutic agents. Semi-crystalline polyurethanes may be resistant to degradation in oxidative, hydrolytic, and enzymatic changes and body fluid contact environments. Higher hardness may provide a polyurethane resistant to swelling and leaching when in contact with CSF, therapeutic agents, and highly concentrated therapeutic agents. Higher hardness polyurethanes may be resistant to degradation in oxidative, hydrolytic, and enzymatic changes and body fluid contact environments. A polyurethane with a higher hardness (i.e. higher A or preferably D hardness scale) may have less soft segment and higher crystallinity, which may slow diffusion and interaction with solvents and solutions. Accordingly, brain catheters comprising polyurethane may be less susceptible to swelling or attack by solvents and solutions of therapeutic fluids.

A brain catheter comprising a polyurethane with higher hardness may allow for reduced wall thickness and may provide for processing advantages for single lumen brain catheters and multi-lumen brain catheters.

In some embodiments, the catheter may be formed from a material having a Shore A hardness in a range from about 70 A to about 110A. In some embodiments, the catheter may be formed from a material having a Shore A hardness above 80 A or preferably above 90A. In some embodiments, the catheter is formed from a material having a Shore D hardness of about 30D to about 70D, such as from about 40D to about 60D, or from about 50D to about 60D.

The brain catheter may comprise a semicrystalline polymers other than a polyurethane. The semicrystalline polymers may be selected to provide resistance to degradation in oxidative, hydrolytic, and enzymatic changes and body fluid contact environments while maintaining the desired flexibility through thinner wall or lower wall thickness. In some embodiments, a brain catheter comprises one or more of polyolefin, polyethylene, a fluorinated polymer, a fluorinated homopolymer, a fluorinated copolymer, a homopolymer of polyvinylidene difluoride (PVDF), a copolymer of PVDF, a copolymer of tetrafluoroethylene (TFE) and hexafluoro propylene (HFP), and polychlorotrifluoroethylene. A polyolefin may provide resistance to change during its *in vivo* life through its high crystallinity and long hydro-carbon chains, and a fluorinated polymer may provide resistance to change during its *in vivo* life through its high crystallinity and the inert nature of fluorine.

Some examples, of polymers that may be used to form a brain catheter as described herein include aliphatic or aromatic, polycarbonate-based thermoplastic polyurethane, such as CARBOTHANE (available from Lubrizol, Wickliffe, Ohio, USA), perfluoroelastomers, such as KALREZ (available from DuPont, Wilmington, Delaware, USA); PVDF, such as KYNAR FLEX (available from Daikin, Osaka, Japan) or KYNAR ULTRAFLEX (available from Arkema, Colombes, France); fluorinated ethylene propylenes, such as NEOFLON (available from Daikin, Osaka, Japan).

It should be understood that identifying a polymer or polymers that provide resistance to substantial breakdown, degradation, erosion, or deterioration of mechanical properties but at the same time is flexible while maintaining catheter dimensions, wall thickness, and structure is not trivial, in particular if the catheter is intended for implanted use over extended periods of time. In addition, it should be understood that the design challenges associated in identifying a suitable polymer or polymers for forming the catheters in combination with design concerns associated with the implantable cranial medical device, some of which are described above, present substantial challenges. Accordingly, the design of a suitable implantable cranial medical device as described herein with a suitable brain catheter was not a trivial task.

In some embodiments multiple single lumen brain catheters are made from the same polymer or polymers. In other embodiments, the multiple single lumen brain catheters are made from different polymers. In some embodiments, multiple single lumen brain catheters are separated from each other. In other embodiments, the two single lumen brain catheters are bonded to each other, such as via chemical bonding or thermal bonding. In some embodiments, more than one lumen of a multi-lumen brain catheter is made from the same polymer or polymers. In other embodiments, lumens of a multi-lumen lumen brain catheter are made from different polymers. In some embodiment, all lumens of a multi-lumen brain catheter are made from the same polymer or polymers. In other embodiments, two or more lumens of a multi-lumen brain catheter are made from two or more different polymers.

Exterior surfaces of the brain catheter preferably comprise material that are biocompatible. Preferably, exterior surfaces of the brain catheter inhibit tissue adhesion. Preferably, exterior surfaces of the brain catheter are easily inserted. A hydrophilic coating, such as a hydrogel, may be applied to an exterior surface to cause the exterior surface to be lubricious to facilitate insertion of the catheter into the brain. The exterior surface of the catheter may be coated with polytetrafluorethylene (PTFE) or another polymer to improve insertability, decrease adhesion, or increase insertability and decrease adhesion. In some embodiments, the structural material of the catheter is sufficiently insertable and sufficiently resists tissue adhesion without an additional coating. If a coating is applied, the surface of the catheter preferably includes functional groups that may covalently bind with a coating. The surface of the catheter may be treated to introduce functional groups, or the functional groups may be present in the material forming the catheter.

The brain catheter may include or may be coated with an antimicrobial material, such as antimicrobial silver or an antibiotic. In some embodiments the brain catheter includes or is coated with a composition comprising a combination of minocycline and rifampin. In some embodiments, the brain catheter is soaked in a solution comprising an antimicrobial agent, and the antimicrobial agent is taken up by the material forming the brain catheter.

The brain catheter may comprise radiopaque material visible by imaging, such as X-ray or magnetic resonance imaging (MRI). The brain catheter may comprise radiopaque material throughout the catheter, may comprise a concentrated area of radiopaque material, or may comprise radiopaque material throughout the catheter and may comprise a concentrated area of radiopaque material. The brain catheter may comprise a concentrated radiopaque material at the distal end portion. The brain catheter may comprise one or more concentrated radiopaque bands or markings along the length of the marker that may be used to determine the depth of the catheter during or after implantation.

The brain catheter may comprise any suitable radiopaque material. Examples of suitable radiopaque material includes barium sulfate, tantalum, and titanium.

In some embodiments, barium sulfate is blended with the polymer forming the catheter such that the barium sulfate is distributed through the catheter. Any suitable concentration of barium sulfate may be used. In some examples, about 5% barium sulfate by weight to about 20% barium sulfate by weight is blended into the polymer forming the catheter. For example, about 10% barium sulfate by weight to about 15% barium sulfate by weight, or about 12% barium sulfate may be blended into the polymer forming the catheter. 12% barium sulfate blended into the polymer was empirically determined to provide a suitable balance of a number of factors, including (i) ability to visualize the catheter throughout the implant process as it penetrates, (ii) manufacturability of thin-walled dual lumen catheter, and (iii) compatibility with fluids comprising high concentration therapeutic agents that may be infused through the catheter. Balancing these factors may achieve biocompatibility and biostability for the life of its implant, such as 5 years or more. In some embodiments, a tantalum marker, such as a tantalum bead, is positioned in the brain catheter at the distal end portion.

The brain catheter may comprise one or more openings in communication with a lumen of the catheter through which fluid may flow. The brain catheter may comprise any suitable number of openings in communication with each lumen. For example, the brain catheter may comprise one to ten or more openings in communication with each lumen, such as two to six openings or three to four openings in communication with each lumen. The brain catheter may have the same number of openings in communication with the first lumen as in communication with the second lumen. The brain catheter may have a different number of openings in communication with the first lumen than in communication with the second lumen.

The openings may be of any suitable size and may be configured in any suitable manner. The openings have a diameter or width of from about 0.2 millimeters to about 1 millimeter, such as from about 0.4 millimeters to about 0.6 millimeters, or about 0.5 millimeters. The openings may have the same or different diameters or widths.

The openings may be any suitable shape. For example, the openings may have circular or elliptical cross-sectional shapes, rectangular cross-sectional shape, triangular cross-sectional shape, or the like, or combinations thereof.

The openings may be always be open or may be configured to open due to a pressure differential in the lumen of the catheter and CSF in which the distal end portion of the brain catheter is implanted. For example, the openings may comprise slits that open due to relative positive pressure in the lumen when fluid is infused through the lumen to the CSF or due to relative negative pressure in the lumen when CSF is aspirated through the lumen. The slit may be cut into a resilient material that flexes when under pressure but returns to an original shape as pressure equalizes.

The openings may be positioned at any suitable location of the catheter. In some embodiments, the one or more openings of the brain catheter in communication a lumen configured to infuse therapeutic fluid (e.g., the lumen in communication with the second fluid path of the implantable cranial medical device) may be positioned a short distance from the distal tip of the brain catheter. The one or more openings of the brain catheter in communication with a lumen through which CSF is configured to be aspirated (e.g., the lumen in communication with the first fluid path of the implantable cranial medical device) may be positioned at or in proximity to the distal tip. Separating infusion openings from aspiration openings may allow for infused fluid to mix with CSF so that aspirated CSF better represents concentrations of therapeutic agent in CSF than if the infusion and aspiration openings were in proximity to each other. If the infusion and aspiration openings are located adjacent to one another, then aspirated CSF may have higher concentration that CSF at a location more remote from the aspiration lumen. If the aspiration and infusion openings are located further apart from one another, then the aspirated fluid would be more representative of the entire CSF.

In addition to positioning aspiration and infusion openings longitudinally apart (closer to or further from the distal tip), the aspiration openings and the infusion openings may be placed on generally opposing sides of the brain catheter. For example, the aspiration openings and the infusion openings may be positioned from about 160 degrees to about 180 degrees radially apart from one another. By positioning the infusion ad aspiration lumens radially apart, substantial mixing of CSF with therapeutic fluid infused through an infusion opening may occur prior to aspirating the CSF through an aspiration opening.

In addition to positioning aspiration and infusion openings away from each other in a staggered manner in the cerebral ventricle, the position of the openings may be substantially separated such that the infusion holes are placed within a tissue location in the brain other than the cerebral ventricle but the aspiration openings remain in the CSF.

The lumens of the brain catheter may have the same or different lengths. The lumens of the brain catheter may have a length that extends from a proximal end portion, which may be coupled to the brain catheter connector of the implantable cranial medical device, to the distal-most opening in the catheter in communication with the respective lumen. If the lumens of the brain catheter are configured to carry fluid to or from the same brain location, such as a cerebral ventricle, the lumens may have the same or substantially similar lengths. If the lumens of the brain catheter are configured to carry fluid to or from different brain locations, such as a ventricle and brain parenchyma, the lumens may be of substantially different lengths. For purposes of the present disclosure, lumens having "substantially different" lengths are lumens that have lengths that differ by more than 10 percent. Lumens that have "substantially similar" lengths are lumens that have lengths that differ by 10 percent or less.

In some embodiments, the brain catheter comprises multiple infusion openings positioned a distance from the distal tip, such as from about 3 millimeters from the distal tip to about 15 millimeters from the distal dip. For example, all the infusion openings may be positioned from about 3 millimeters to about 10 millimeters from the distal dip, or from about 4 millimeters to about 8 millimeters from the distal tip. In some embodiments, at least one aspiration lumen is positioned at the distal tip of the brain catheter.

In some embodiments, the implantable cranial medical device comprises a catheter connector configured to connect to an external catheter. For purposes of the present disclosure, an "external catheter" is a catheter that may be coupled to a device separate from the implantable cranial medical device or may serve as a CSF shunt or drainage catheter. The "external catheter" may be completely implanted in a subject when in use or may be partially implanted and partially external to the subject when in use. The separate device may be, for example, an infusion device. The infusion device may be implantable or non-implantable. The non-implantable infusion device may be an ambulatory device or a stationary device. The infusion device may be manually powered, electromechanically powered, chemically powered, or otherwise powered. In some examples, the infusion device may comprise a piston pump, a peristaltic pump, an osmotic pump, a plunger, or the like. A distal portion of the CSF shunt or drainage catheter may be placed in any suitable location of the patient, such as the peritoneal cavity.

The external catheter connector of the implantable cranial medical device may be coupled to the catheter in any suitable manner. For example, the external catheter and the external catheter connector may comprise quick-release couplings, luer lock fittings, snap connect couplings, or the like. In some examples, the external catheter is configured to be coupled to the external catheter connector via interference fit. The external catheter connector may comprise a fitting, which may comprise external barbs, configured to be inserted into a lumen of the external catheter to retain the external catheter relative to the external catheter connector and to place the lumen of the external catheter in fluid communication with the fluid flow path of the implantable cranial medical device.

In some embodiments, the external catheter connector comprises a first portion of a compression fitting and a second portion of the compression fitting is placed over the catheter. Connecting the first and second portion of the compression fitting may compress the external catheter to secure the catheter to the external catheter connector. For example, the compression fitting may cause the catheter to compress against external barbs. The second portion of the compression fitting may comprise a compression ferrule and a connection element to connect to the first portion of the fitting on the external catheter connector.

The catheter connector may be configured to securely engage any suitable catheter. In some embodiments, the external catheter connector is configured to couple to a catheter having an inner diameter from about 0.4 millimeters to about 1 millimeter, such as about 0.5 millimeters to about 0.7 millimeters, or about millimeters.

The external catheter connector may be positioned at any suitable location of the implantable cranial medical device. Preferably, the external catheter connector is located at a portion of the device that is implanted between the skull and the scalp. For example, the external catheter connector may be formed from, operably coupled to, or formed from and operably coupled to an opening in the upper flange portion.

The external catheter connector may be oriented in any suitable manner. For example, the external catheter connector may have a longitudinal axis that extends substantially parallel to a bottom surface of the upper flange portion. For purposes of the present disclosure, a longitudinal axis that extends "substantially" parallel to a surface is a longitudinal axis that does not vary from parallel by more than 10 degrees. As another example, the housing, when defining an exterior surface of the upper flange portion and the lower portion, may comprise a central axis that extends through the lower portion and the upper flange portion, and the external catheter connector may have a longitudinal axis that extends substantially orthogonal to the central axis. For purposes of the present disclosure, a longitudinal axis that extends "substantially" orthogonal to another axis is a longitudinal axis that does not vary from orthogonal by more than 10 degrees. Preferably, the external catheter connector extends substantially parallel to the skull of the patient. Such an orientation may permit the catheter to readily run along a surface of the skull in proximity to the implantable cranial medical device.

In some embodiments, the implantable cranial medical device comprises a port defining an opening in communication with a fluid flow path of the implantable cranial medical device. The port is preferably configured to receive a needle through which fluid may be infused into, or withdrawn from, the fluid path of the device. Preferably, the port is positioned and oriented to allow the needle to access the port through the scalp of the subject in which the device is implanted. For example, the port may be defined by, or operably coupled to, a top surface of the device, such as the top surface of the upper flange portion of the housing. Preferably, the port is substantially aligned with the center of the top surface of the upper flange portion of the housing.

The port may comprise a ferrule or funnel that decreases in inner diameter from a location in proximity to the top surface of the device to an interior portion of the device to facilitate alignment with the needle inserted in the port with a portion of the fluid flow path. The ferrule or funnel may also limit insertion of the needle beyond a bottom of the ferrule or funnel. The ferrule or funnel may be formed from any suitable material, such as a metallic material, a ceramic material, a glass material, or a hard plastic material, or combinations thereof.

The port may comprise a sealing element, such an O-ring, to seal the needle relative to the port and to place a lumen of the needle in sealed communication with the fluid flow path of the implantable cranial medical device.

A septum may be disposed across the opening of the port. Preferably, the septum may be a self-sealing septum. A self-sealing septum may allow multiple cycles of needle insertion into and withdrawal from the port while continuing to seal the port from an interstitial environment in which the port may be located when implanted. The implantable cranial medical device may comprise any suitable self-sealing septum. For example, the self-sealing septum may comprise silicone, a polyethylene, of the like, and combinations thereof.

The port may be configured to receive a needle of any suitable gauge. The needle may be inserted through the septum to infuse fluid through the first fluid flow path and first lumen of a catheter communication with the first fluid flow path or to aspirate fluid from the subject through the first lumen and first fluid flow path. The tapered nature of the ferrule or funnel may accommodate needles of a variety of sizes. For example, the ferrule or funnel may accommodate needles with a range of diameter sizes from about 0,63 mm to 1,7 mm (16 gauge to 25 gauge), such as from about 0,9 mm to 1,3 mm (18 gauge to 22 gauge). The gauge of needle employed may vary depending on the material introduced or withdrawn from the port. For example, a smaller gauge needle may be more desirable for more viscous fluids of fluids containing larger molecules or particles, such as cells or proteins.

Preferably, port is configured to receive non-coring needles, such as Huber needles or butterfly needles. Non-coring needles may be designed with a deflected or offset 'B' bevel point. Such a tip has the advantage of parting rather than cutting a plug from or coring the septum of the port and may create a more comfortable injection. Using a non-coring needle, such as a Huber needle, may preserve the integrity of the septum and may prevent a plug of septum material from being cut and passed into the CSF.

In some embodiments, a kit may include one or more non-coring needles.

The ferrule or funnel may have an inner surface defining a reservoir. The reservoir may have any suitable volume. For example, the reservoir may have a volume suitable to receive a non-coring needle. In some embodiments, the reservoir has a volume within a range from about 2 milliliters to about 7 milliliters, such as from about 3 milliliters to about 5 milliliters.

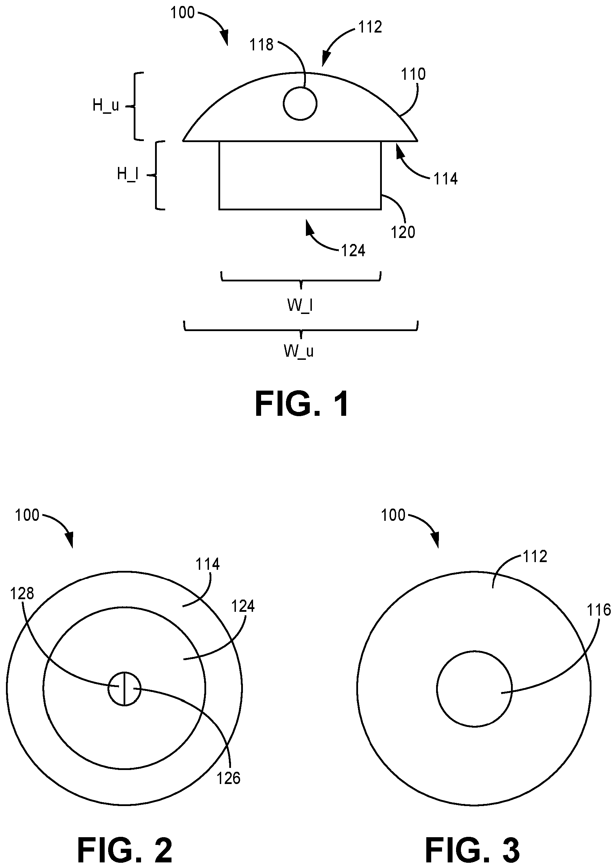

Reference is now made to **FIGS. 1-3****,** which illustrate an example of an embodiment of an implantable cranial medical device **100** of the present disclosure. The implantable cranial medical device **100** comprises an upper flange portion **110** and a lower portion **120.** The upper flange portion has a generally convex top surface **112** and a generally flat bottom surface **114.** The upper flange portion **110** is configured to be positioned between a skull and a scalp of a subject when implanted, with the bottom surface **114** configured to rest on a skull of a subject. The upper flange portion **110** has a height **H_u** and a width **W_u.** The height **H_u** of the upper flange portion **114** is sufficiently small to avoid skin erosion and substantial discomfort to the subject when implanted. The width **W_u** of the upper flange portion **114** is sufficiently large to rest on the skull around a burr hole.

The lower portion **120** has a bottom surface **124,** a height **H_l** and a width **W_l.** The height **H_l** of the lower portion **120** is sufficiently small so that the bottom major surface **124** of the lower portion **120** does not extend substantially below the burr hole when implanted in a subject. Preferably, the bottom major surface **124** of lower portion **120** does not extend below the burr hole when implanted in a subject. The width **W_l** of the lower portion **120** is sufficiently small such that the lower portion **120** fits within the burr hole when implanted.

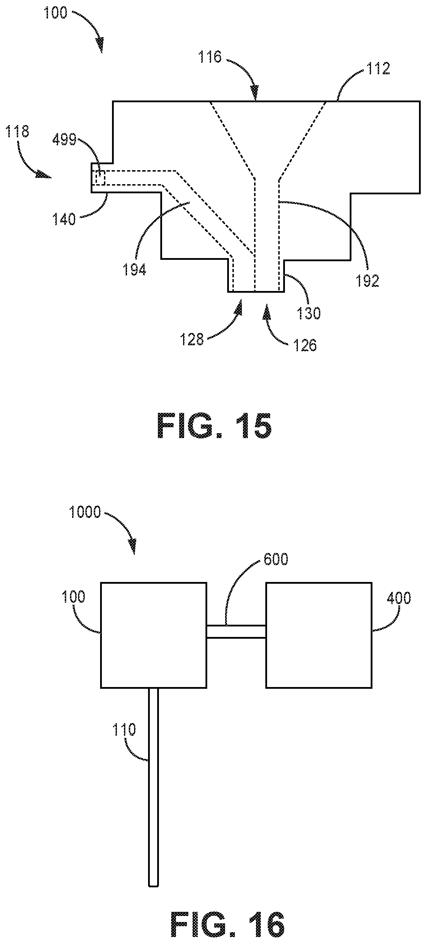

The upper flange portion **110** defines a first opening **116** and a second opening **118.** The lower portion **120** defines a first opening **126** and a second opening **128.** A first fluid flow path (not shown in **FIGS. 1-3**) extends within a housing **101** from the first opening **116** of the upper flange portion **110** to the first opening **126** of the lower portion **120.** A second fluid flow path (not shown in **FIGS. 1-3**) extends within the housing **101** from the second opening **118** of the upper flange portion **110** to the second opening **128** of the lower portion **120,** as shown in Fig.15.

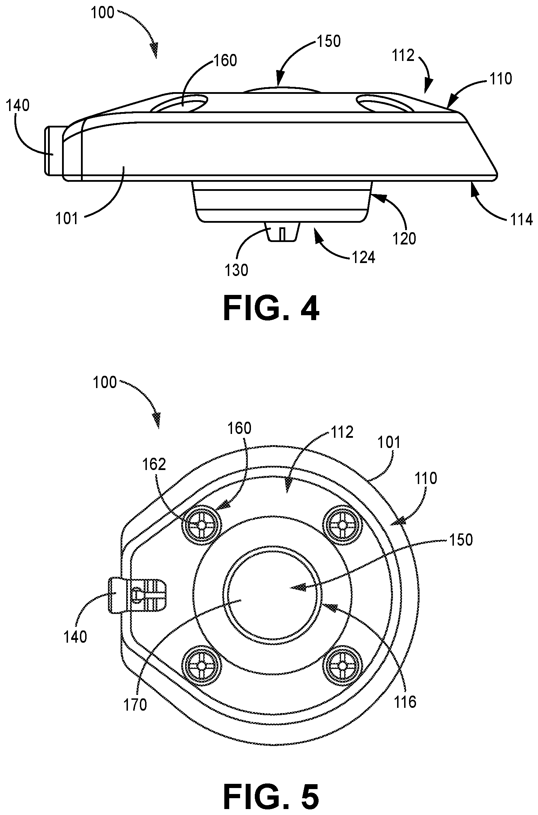

Reference is now made to **FIGS. 4-7** in which an embodiment of an implantable cranial medical device **100** is shown. As with the embodiment shown in **FIGS. 1-3****,** the embodiment of an implantable cranial medical device **100** shown in **FIGS. 4-7** includes an upper flange portion **110** having a top surface **112** and a bottom surface **114** and includes a lower portion **120** having a bottom major surface **124.** Fastener feedthroughs **160** extend through the upper flange portion **110** from the top surface **112** to the bottom surface **114** and are configured to receive fasteners **162,** such as screws, which may be used to anchor the housing to the skull of a subject in which the device **100** is implanted.

The device **100** includes a port **150** in communication with the first fluid path **192.** The lateral edge of the port **150** is defined by the first opening **116** defined by the top surface **112** of the upper flange portion **110.** A self-sealing septum **170** extends across the opening **116.** When the device **100** is implanted, the port **150** is positioned and oriented to receive a needle inserted through the scalp such that a lumen of the needle is placed in fluid communication with the first fluid path **192.**

The device **100** includes a reservoir **180** in communication with and forming a part of the first fluid path **192.** The reservoir **180** is positioned such that insertion of a needle into the port **150** places a lumen of the needle in fluid communication with the reservoir **180.** The reservoir **180** is disposed with the housing **101** and may form a part of the first fluid flow path **192.**

The device also includes an external catheter connector **140** that extends substantially parallel with the bottom surface **114** of the upper flange portion **110** of the device **100.** The external catheter connector **140** is operably coupled to an opening defined by the upper flange portion **110** (such as second opening **118** depicted in **FIG. 1**). The external catheter connector **140** defines a passageway in communication with the second fluid path **194** of the device **100.** The external catheter connector **140** is configured to operably couple to an external catheter to place a lumen of the external catheter in fluid communication with the second fluid path **194.** The external catheter may further be coupled to a device other than the implantable cranial infusion device, such as an infusion device. The infusion device may implantable or non-implantable.

The implantable cranial medical device **100** also includes a brain catheter connector **130** having a first lumen **132** and a second lumen **134.** The brain catheter connector **130** extends from the bottom major surface **124** of the lower portion of the housing **120.** The first lumen **132** of the brain catheter connector **130** is operably coupled to the first fluid path **192.** The second lumen **134** of the brain catheter connector **130** is operably coupled to the second fluid path **194.** The brain catheter connector **130** is configured to operably couple to a dual lumen catheter, which may have a distal end. The distal end may be implantable in a CSF-containing space, such as a cerebral ventricle.

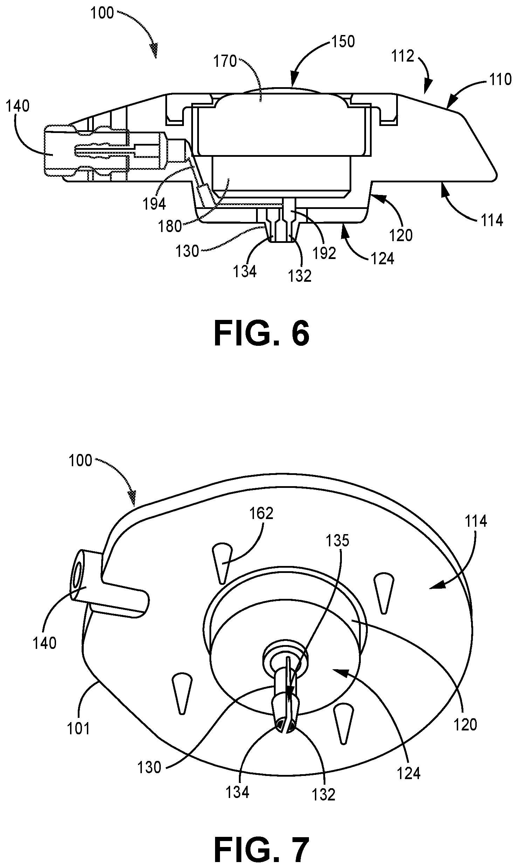

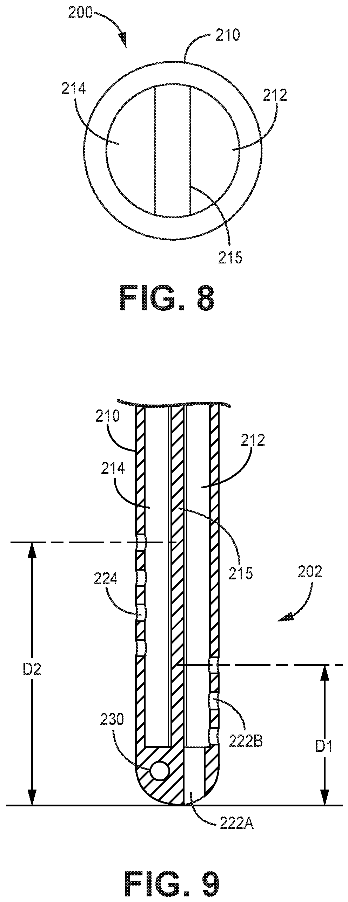

As shown in **FIG. 7****,** the brain catheter connector **130** may include a slot **135** configured to receive a portion of a catheter that separates one lumen from another in a dual catheter. For example and with reference to **FIG. 8****,** a central portion **215** of a body **210** of a brain catheter **200** that separates a first lumen **212** from a second lumen **214** may be inserted into slot **135** of the brain catheter connector **130** such that the first lumen **212** of the catheter **200** is placed in fluid communication with the first lumen **132** of the brain catheter connector **130** and such that the second lumen **214** of the catheter **200** is placed in fluid communication with the second lumen **134** of the brain catheter connector **130.**

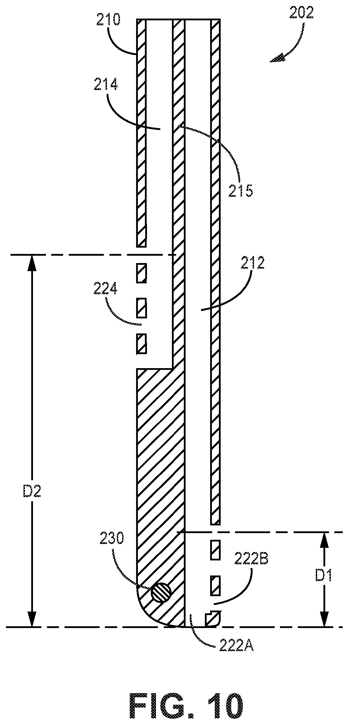

Referring now to **FIGS. 9** and **10****,** distal portions **202** of embodiments of brain catheters are shown. The brain catheter includes a first lumen **212** and a second lumen **214** separated by a central wall **215** of the body **210** of the catheter. The first lumen **212** is in communication with one or more openings **222** at the distal end portion **202.** In the depicted embodiment, the first lumen **212** is in communication with three side openings **222B** at the distal end portion **202** and a distal end opening **222A.** The second lumen **214** is in communication with one or more side openings **224** at the distal end portion **202.** In the depicted embodiment, the second lumen **212** is in communication with four side openings **224** at the distal end portion **202.** The side openings **222A** and **224** are positioned on generally opposing sides of the catheter. The distance (**D1**) from the distal tip to the side opening **222A** furthest from the distal tip is less than the distance (**D2**) from the distal tip to the side opening **224** furthest from the distal tip. Preferably, all the openings **224** in communication with the second lumen **214** are positioned away from the distal tip a distance greater than **D1.** The position and orientation of the openings **222A, 222B, 224** allow for therapeutic fluid that may be infused through the second lumen through side openings **224** to substantially mix with CSF prior to being aspirated through openings **222A, 222B** and through the first lumen **215.**

In the embodiment depicted in **FIG. 10**, **D2** is substantially greater than **D1.** Accordingly, the catheter depicted in **FIG. 10** may be suitable for delivering fluid to, or withdrawing fluid from, for example, a cerebral ventricle through openings **222A** and **222B** and may be suitable for delivering fluid to brain parenchyma through openings **224.**

In the embodiment depicted in **FIG. 9****,** the difference between **D2** and **D1** is not as larger as depicted in **FIG. 10****.** Accordingly, the catheter depicted in **FIG. 9** may be suitable for delivering fluid to, or withdrawing fluid from, for example, a cerebral ventricle through openings **222A** and **222B** and through openings **224.**

In the embodiment depicted in **FIGS. 9** and **10****,** the catheter includes a radiopaque marker **230** visible by X-ray, magnetic resonance imaging (MRI), or X-ray and MRI imaging so that the location of the distal end portion **202** may be readily determined. The depicted radiopaque marker **230** comprises tantalum and is embedded in the distal end of the catheter. The body **210** may comprise barium sulfate or another radiopaque material (not shown) dispersed throughout. In some embodiments (not shown), the brain catheter may include radiopaque markers at locations along the length of the catheter to form depth markings that may be used to determine the depth of the catheter during or after implantation.

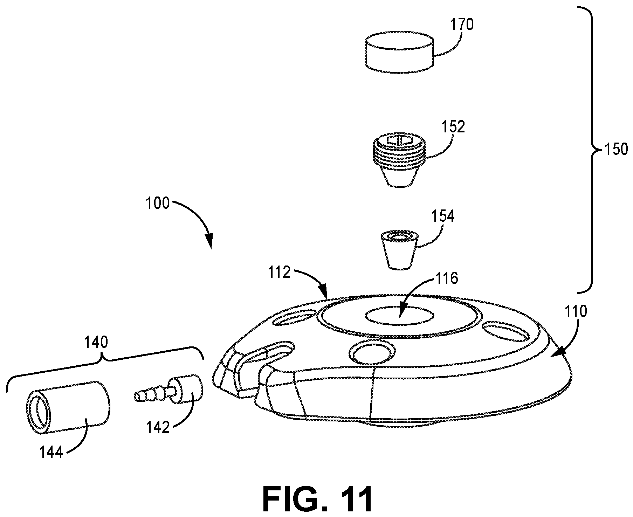

Referring now to **FIG. 11****,** components of an external catheter connector **140** and a port **150** associated with an upper flange portion **110** of an implantable cranial medical device **100** are shown. The outer edge of the port **150** is defined by a first opening **116** defined by a top surface **112** of the upper flange portion **110.** The port **150** includes a septum **170** or cap, a compression wedge **152,** and an axial ferrule **715,** which defines a decreasing inner diameter moving in a direction from the top surface **112** of the upper flange portion **110** towards the bottom surface of the upper flange portion **110.** The axial ferrule **715** serves to guide a needle inserted into the port **150** into a position suitable for placing a lumen of the needle in fluid communication with the first fluid path of the device **100.** The axial ferrule **715** may also limit insertion of the needle beyond a bottom end of the ferrule **715.**

The external catheter connector **140** includes a compression fitting, such as sleeve **144,** defining a lumen configured to receive an external catheter and a barbed fitting **142** configured to be inserted into a lumen of the external catheter. The barbed fitting **142** includes a passageway in communication with the second fluid path of the device **100.** The compression sleeve **144** may be disposed about the external catheter and slid towards the end of the catheter after the catheter has been advanced over the barbed fitting **142** to cause the external catheter to be compressed against the barbed fitting **144.**

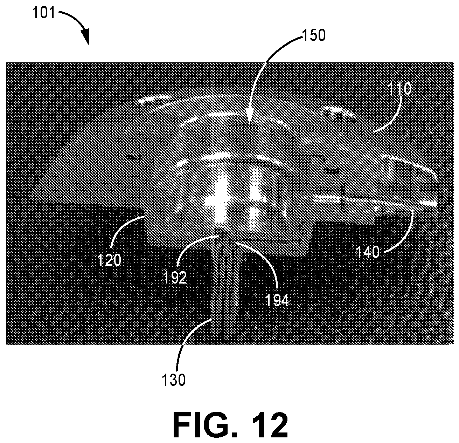

Referring now to **FIG. 12****,** a photograph of a cut-away of a housing **101** of an embodiment of implantable cranial medical device is shown. The housing **101** defines the upper flange portion **110,** the lower flange portion **120,** the external catheter connector **140,** the brain catheter connector **130,** the first fluid path **192,** and the second fluid path **194.** The housing **101** also defines an opening **199** configured to components of a port, such as a septum and reservoir to guide a needle in position relative to the first fluid flow path **192.** The depicted housing **101** is formed from titanium but may be formed from any suitable material.

### Kit

The implantable cranial medical device described herein may be included in a kit. The kit may include a brain catheter. The brain catheter may be coupled to the implantable cranial medical device in the kit or may be coupled by an end user, such as a healthcare professional. Preferably, the brain catheter is coupled to the implantable cranial medical device prior to packing in the kit. The kit may include a compression fitting to aid in connecting an external catheter to an external catheter connector of the implantable cranial medical device. The kit may include fasteners, such as screws, configured to secure the upper flange portion of the implantable cranial medical device to the skull of a subject. The kit may include a tool, such as a screwdriver, for use with the fasteners. The kit may include a needle configured to access a port of the implantable cranial medical device, which may be used for, for example, aspiration. The needle may be a non-coring needle, such as a Huber needle or a butterfly needle.

While only a few kit components are listed above, it will be understood that any component discussed herein may be included in a kit comprising an implantable cranial medical device.

### Implantation (does not form part of the invention)

The implantable cranial medical devices and associated devices described herein may be implanted in any suitable manner. In some embodiments, a brain catheter having a distal end and a proximal end is implanted such that the distal end is positioned in a CFS-containing region, such as a cerebral ventricle, of a subject. The proximal end may be positioned in proximity to a burr hole in a skull. Preferably, the brain catheter is a dual lumen catheter. The proximal end of the catheter may be coupled to the implantable cranial infusion device to place one lumen of the brain catheter in communication with the first fluid path of the implantable cranial medical device and the second lumen of the brain catheter in communication with the second fluid path of the implantable cranial medical device. The implantable cranial infusion device may be implanted such that the lower portion is disposed in the burr hole and the upper flange portion is between a skull and a scalp of a subject. The upper flange portion may be secured to the skull via one or more fasteners, such as screws.

The brain catheter may be coupled to the implantable infusion device before or after the distal end of the brain catheter is positioned in the cerebral ventricle. Preferably, the brain catheter is coupled to the implantable cranial medical device prior to the brain catheter being implanted. A stylet may be inserted through a fluid flow path, such as the first fluid flow path, of the implantable cranial medical device and into a lumen, such as the first lumen, of the brain catheter to facilitate implanting the catheter into the brain of a subject. For example, the stylet may be inserted through a septum of a port in communication with the first fluid path, through the first fluid path, and into the first lumen.

A subject, in which the implantable cranial medical device and associated components may be implanted, may undergo a preoperative scan for surgical planning. A surgical navigation system may be employed during an implant procedure to facilitate placement of the brain catheter. The brain catheter may be radiopaque, may include one or more radiopaque markers, or may be radiopaque and include one or more radiopaque markers to allow visualization with the surgical navigation system employed. For example, Medtronic, Inc.'s AxiEM^{™} electromagnetic technology and StealthStation^{®} Navigation System and B. Braun Aesculap division's Intraventricular Disposable Introducer Set^{™} may be used to facilitate implantation of the catheter. Preferably, the distal end portion of the catheter is positioned in a cerebral ventricle of the subject. Preferably, the distal end portion of the catheter is positioned in a lateral ventricle. However, the distal end portion of the brain catheter may be placed in any suitable location, such in the cisterna magna, a subarachnoid space, or the like.

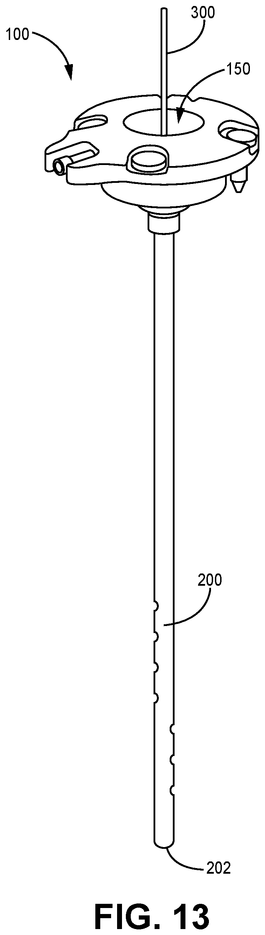

Referring now to **FIG. 13****,** an example illustrating an implantable cranial medical device 100 and coupled brain catheter **200** is show. The device **100** and brain catheter **200** are shown prior to the catheter **200** being implanted. As shown in **FIG. 13****,** a stylet **300** may be inserted into the port **150** of the implantable cranial medical device **100,** through the first fluid path (such as fluid path **192** shown in **FIG. 6**) and at least partially through a first lumen (such as lumen **212** shown in **FIG. 8**) to facilitate implanting the distal end **202** of the catheter **202** in a cerebral ventricle of a subject. The brain catheter **200** is typically flexible and lacks rigidity. The stylet 300 may provide structural rigidity to allow insertion of the distal end **202** of the catheter **200** through brain tissue and into the cerebral ventricle.

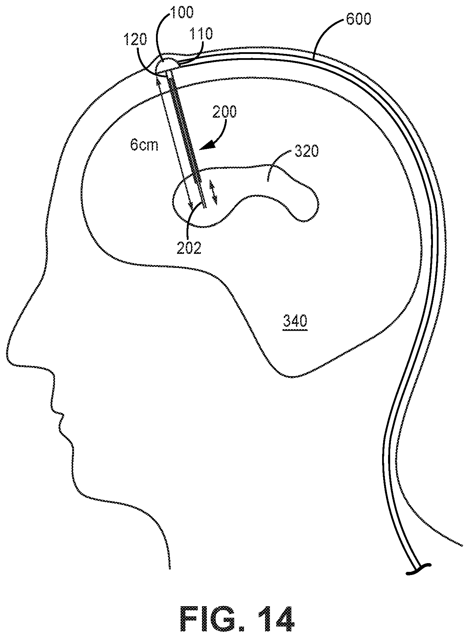

For example, and as shown in **FIG. 14****,** the distal end portion **202** of the brain catheter **200** may be implanted in a lateral ventricle **400** of a brain **500** of a subject. The proximal end of the brain catheter **200** may be coupled to a brain catheter connector (not shown) of the device **100.** The lower portion **120** of the implantable cranial medical device **100** may be positioned within a burr hole (not shown) of a skull (not shown) of the subject, and the upper flange portion **110** of the device **100** may be implanted between the skull and the scalp of the subject. An external catheter **600** may be coupled to an external catheter connector (not shown) of the implantable cranial medical device **100** and positioned along the skull of the subjected and tunneled to a position in the patient remote from the implantable cranial medical device **100.** For example, the external catheter **600** may be operably coupled to an infusion device implanted subcutaneously in a location of a torso, such as in the abdomen region, of the subject.

The brain catheter **300** may have any suitable length, such as about 6 centimeters to about 7 centimeters, which should be sufficient to connect to the implantable cranial medical device **100** and extend into the lateral ventricle **400** of the brain **500** of the subject.

### Use (does not form part of the invention)

The implantable cranial medical devices and associated devices described herein may be used in any suitable mater. If the distal end portion of the brain catheter is positioned in a CSF-containing space of the subject, the implantable cranial medical device may be permit infusion of therapeutic fluid and aspiration of CSF through separate lumens of the brain catheter and through separate fluid paths of the device.

For example, fluid may be aspirated from the CSF-containing space of the subject through the first lumen of the brain catheter and through the first fluid path of the implantable cranial medical device. A lumen of a needle may be placed in communication with the first fluid path of the implantable cranial medical device. For example, the needle may be inserted into a port in communication with the first fluid path. The fluid may be aspirated from the CSF-containing space of the subject through lumen of the needle.

In some embodiments, the first fluid path of the implantable cranial medical device and the first lumen of the brain catheter may be used to infuse fluid, such as a fluid comprising a therapeutic agent, into the CSF-containing space of the subject. For example, a lumen of a needle may be placed in communication with the first fluid path of the implantable cranial medical device. For example, the needle may be inserted into a port in communication with the first fluid path. The fluid may be infused through the lumen of the needle, through the first fluid path of the implantable cranial medical device, and through the first lumen of the brain catheter to the CSF-containing space of the subject.

A fluid may be infused to the CSF-containing space of the subject by infusing the fluid through the second flow path of the implantable cranial medical device and through the second lumen of the brain catheter. The fluid may comprise a therapeutic agent. An infusion device may be coupled to the second fluid path of the implantable cranial medical device, such as via an external catheter, and the fluid may be infused from the infusion device to the CSF-containing space. The infusion device may be an implanted infusion device.

By providing separate lumens and fluid paths, infusion and aspiration (or infusion) may be conducted simultaneously. Accordingly, infusion of a first therapeutic fluid does not need to be disrupted to aspirate a sample of CSF or to introduce a second therapeutic fluid to the CSF. Such lack of disruption of infusion of the first therapeutic fluid may provide for improved therapy.

The devices, kits, and systems described herein may extend the space and time a therapeutic agent is available to its brain target improving pharmacokinetics and pharmacodynamics of the therapeutic agent in the brain relative to prior approaches for direct infusion into the central nervous system.

CSF aspirated via the first fluid path may be used for any suitable purpose. For example, aspirated CSF may be used to determine the concentration of a therapeutic agent, which may be introduced via a therapeutic fluid infused through the second fluid path to the CSF. The flow rate or pattern of infused therapeutic may be adjusted based on concentrations of therapeutic agent determined to be present in the aspirated CSF.

The aspirated CSF may be used to determine whether the subject may have an infection due to implantation or use of the implantable cranial medical device and associated devices. If, for example, infectious bacteria, fungi, or viruses are detected in the aspirated CSF, antibiotics, anti-fungal agents, or antiviral agents may be administered to the CSF through, for example the first fluid flow path of the cranial medical device and first lumen of the brain catheter. Concentration or presence of infectious pathogens in subsequently aspirated CSF may be used to determine whether parameters of therapy should be adjusted or whether the cranial medical device and associated devices should be explanted.

The aspirated CSF may be used to detect or diagnose serious bacterial, fungal and viral infections, including meningitis, encephalitis and syphilis. The aspirated CSF may be used to detect or diagnose bleeding around the brain (subarachnoid hemorrhage). The aspirated CSF may be used to detect or diagnose certain cancers involving the brain or spinal cord. The aspirated CSF may be used to detect or diagnose certain inflammatory conditions of the nervous system, such as multiple sclerosis and Guillain-Barre syndrome.

For example, normal CSF white blood cell count is between 0 and 5, and normal CSF red blood cell count is 0. An increase of white blood cells may indicate infection or inflammation, an increase in red blood cell count may indicate bleeding into the cerebrospinal fluid.:

The aspirated CSF may be used to determine whether the presence or concentration of a biological marker associated with the disease being treated changes in response to the infused therapeutic fluid. In fact, all diseases of the central nervous system have a CSF profile that is unique to the disease and to the progression of the disease. For ALS, markers may include a change in one or more molecules associated with inflammation. For Alzheimer's disease markers may include beta amyloid and tau. For Huntington's disease, a marker may include the hunitingtin protein. For Parkinson's disease, markers may include tau and leucine-rich repeat kinase 2 (LRRK-2).

The aspirated CSF may be used to determine the presence of biomarkers associated with diagnosis and prognosis. Such biomarkers include microRNA such as miR-146a and miR-134 as biomarkers for epilepsy diagnosis and prognosis. The association of miR-146a and miR-134 and epilepsy is described in, for example, Leontariti, et al., "Circulating miR-146a and miR-134 in predicting epilepsy in patients with focal impaired awareness seizures," Epilepsia, May 2020 (e-published April 21, 2020); 61(5) 959-970.

As another example, a seizure event may cause a change in the CSF profile of an individual. More specifically, immunoglobulin synthesis, elevated lactate, cell count, glucose and total protein concentrations as well as blood-brain barrier dysfunction are frequently observable in the CSF profile following epileptic seizures. Blood cell count may be a marker indicative of seizure disorder. For example, higher blood cell counts may be indicative of more severe disorder and often longer hospital stays.

The infusion parameters or other parameters of therapy may be changed based on the presence of concentration of the biological marker in the aspirated CSF.

The aspirated CSF may be used to determine whether the presence or concentration of a biological marker indicative of toxicity of a therapeutic fluid infused into the CSF (*e.g.,* via the second fluid path and second lumen of the catheter). For example, the presence of antibodies or other inflammatory response to the infused therapeutic agent may be indicative of potential toxicity of the infused therapeutic agent. The presence or increased concentrations of one or more of S100 proteins, neuron specific enolase (NSE), tau, and beta-amyloid may be indicative of toxicity. If a biomarker indicative of toxicity is detected in aspirated CSF, the rate of infusion of therapeutic fluid may be reduced or infusion of the therapeutic fluid may be discontinued.

In some embodiments, intracranial pressure may be determined by placing a pressure monitor in fluid communication with the first fluid path of the implanted cranial medical device. For example, a needle operably coupled to the pressure sensor may be placed in the port to determine intracranial pressure.

In some embodiments, aspirated CSF combined with therapeutic agents and the infused back into the CSF-containing space.

In some embodiments, a dye or radioactive substances (ventriculograpy, cisternography) may be infused into cerebrospinal fluid to facilitate the making of diagnostic images of the fluid's flow within a subject's brain.

Any suitable therapeutic fluid may be infused through the first or second fluid path of the implantable cranial medical device and the first of second lumens of the brain catheter. The therapeutic fluid may comprise any suitable therapeutic agent. Preferably, the therapeutic agent is an agent for treating a disease of the brain. Examples of diseases of the brain include any diseases with pathology or dysfunction occurring in any component of the brain (including the cerebral hemispheres, diencephalon, brain stem, and cerebellum) or the spinal cord. Examples include but are not limited to Parkinson's disease, Alzheimer's disease, dementia, Amyotrophic Lateral Sclerosis, Huntington's disease, lysosomal storage diseases, post-traumatic stress disorder, anxiety, depression, brain tumors, autism, autism spectrum disorder, closed head injury, spinal cord injury, stroke, multiple sclerosis, schizophrenia, anxiety, and epilepsy. Preferably, the implantable cranial medical device and associated devices are used to treat a disease of the brain that is resistant to treatment through systemic routes of administration, such as oral, intravenous, intramuscular, and intraperitoneal administration. The implantable cranial medical device and associated devices may also be used if a patient is at serious risk if direct central administration of a therapeutic fluid is not commenced.

The therapeutic fluid may be infused into the CSF or other brain region at any suitable rate. Preferably, flow rate into the brain is limited to 20 milliliters or less per day, such as 10 milliliters or less per day, or 5 milliliters per day or less. For example, the therapeutic fluid may be infused at a metered rate of 4 milliliters per day or less, such as 3 milliliters per day or less, 2 milliliters per day or less, or about 1 milliliter per day.

Withdrawal of CSF from the brain should be limited to 3,500 milliliters per day or less, preferably 200 milliliters per day or less.

The therapeutic fluid may comprise any suitable therapeutic agent. The therapeutic agent selected may depend on the disease being treated. The therapeutic fluid, such as a solution, may contain any suitable concentration of the therapeutic agent. The concentration of the therapeutic agent will depend on the therapeutic agent employed. In some embodiments, the therapeutic fluid is a solution comprising a therapeutic agent at a concentration in a range of from about 10 milligrams per milliliter to about 500 milligrams per milliliter, such as from about 50 milligrams per milliliter to about 450 milligrams per milliliter.

For purposes of illustration, a list of suitable anti-epileptic therapeutic agent that may be included in a therapeutic fluid, such as a solution, includes carbamazepine; tiagabine, levetiracetam; lamotrigine; pregabalin; fenfluramine; gabapentin; phenytoin; topiramate; oxcarbazepine; valproate; valproic acid; zonisamide; perampanel; eslicarbazepine acetate; lacosamide; vigabatrin; rufinamide; fosphenytoin; ethosuximide; phenobarbital; diazepam; lorazepam; clonazepam; clobazam; ezogabine; felbamate; primidone; acetazolamide; brivaracetam; clorazepate; ethotoin; mephenytoin; methsuximide; trimethadione; bumetanide; adenosine; and an adenosine a1 receptor agonist. In some embodiment, the therapeutic agent is valproic acid or a pharmacologically acceptable salt thereof. For purposes of the present disclosure, reference to a compound includes reference to salts, hydrates, solvates, and polymorphs thereof.

Examples of other therapeutic agents that may be delivered using an implantable cranial medical device for treating or diagnosing a CNS disease include Edaravone (e.g., Radicava^{®}) for Amyotrophic Lateral Sclerosis (ALS), Valbenazine (e.g., Ingrezza^{®}) for Tardive dyskinesia, Deuterabenazine (e.g., Austedo^{®}) for Huntington's disease, Ocrelizumab (e.g., Ocrevus^{®}) for Multiple sclerosis, Safinamide (e.g., Xadago^{®}) for Parkinson's disease, Nusinersen (e.g., Spinraza^{®}) for Spinal muscular atrophy (SMA), Daclizumab (e.g., Zinbryta^{®}) for Multiple sclerosis, Pivavanserin (e.g., Nuplazid^{®}) for Hallucinations and delusions associated with psychosis, Ariprprazole lauroxil (e.g., Aristada^{®}) for Schizophrenia, Caripazine (e.g., Vraylar^{®}) for Schizophrenia and bipolar disorder, Brexpiprazole (e.g., Rexulti^{®}) for Schizophrenia, Peginterferon beta-1a (e.g., Plegridy^{®}) for Multiple sclerosis, Eslicarbazepine acetate (Aptiom^{®}) for Epilepsy associated seizures, Flutemetamol F 18 (e.g., Vizamyl^{®}) Radioactive diagnostic for Alzheimer's disease, Vortioxetine (e.g., Brintellix^{®}) for Major depressive disorder, Dimethyl fumerate (e.g., Tecfidera^{®}) for multiple sclerosis, and Gadoterate megumine (e.g., Dotarem^{®}) for MRI-based brain imaging.

Examples of other therapeutic agents that may be delivered using an implantable cranial medical device as described herein include drugs and biologics such as Coagulation Factors, Cytokines, Epigenetic protein families, Growth Factors, Hormones, Peptides, Signal Transduction molecules, and mutations thereof; also including Amino Acids, Vaccines and/or combinations thereof. Therapeutic compounds further include antibodies, antisense, RNA interference made to the above biologics and their target receptors and mutations of thereof. Additional therapeutic compounds include Gene Therapy, Primary and Embryonic Stem Cells. Also included in the therapeutic compounds are antibodies, antisense, RNA interference to Protein Kinases, Esterases, Phosphatases, Ion channels, Proteases, structural proteins, membrane transport proteins, nuclear hormone receptors and/or combinations thereof.

Examples of Coagulation Factors include, but are not limited to: Fibrinogen, Prothrombin, Factor I, Factor V, Factor X, Factor VII, Factor VIII, Factor XI, Factor XIII, Protein C, Platelets, Thromboplastin, and Co-factor of VIIa.

Examples of Cytokines include, but are not limited to: Lymphokines, Interleukins, Chemokines, Monokines, Interferons, and Colony stimulating factors.

Examples of Epigenetic protein families include, but are not limited to: ATPase family AAA domain-containing protein 2 (ATAD2A), ATPase family-AAA domain containing 2B (ATAD2B), ATPase family AAA domain containing-2B (ATAD2B), bromodomain adjacent to zinc finger domain-1A (BAZ1A), bromodomain adjacent to zinc finger domain-1B (BAZ1B), bromodomain adjacent to zinc finger domain-2A (BAZ2A), bromodomain adjacent to zinc finger domain-2A (BAZ2A), bromodomain adjacent to zinc finger domain-2B (BAZ2B), bromodomain-containing protein 1 (BRD1), Bromodomain containing protein 2-1st bromodomain (BRD2), Bromodomain containing protein 2-1st & 2nd bromodomains (BRD2), bromodomain-containing protein 2 isoform 1-bromodomain 2 (BRD2(2)), bromodomain-containing protein 3-bromodomain 1 (BRD3(1)), Bromodomain-containing protein 3-1st bromodomain (BRD3), Bromodomain-containing protein 3-1st & 2nd bromodomains (BRD3), bromodomain-containing protein 3-bromodomain 2 (BRD3(2)), Bromodomain containing protein 4-1st bromodomain (BRD4), bromodomain-containing protein 4 isoform long-bromodomains 1 and 2 (BRD4(1-2)), bromodomain-containing protein 4 isoform long-bromodomain 2 (BRD4(2)), bromodomain-containing protein 4 isoform short (BRD4(full-length-short-iso.)), Bromodomain containing protein 7 (BRD7), bromodomain containing 8-bromodomain 1 (BRD8(1)), bromodomain containing 8-bromodomain 2 (BRD8(2)), bromodomain-containing protein 9 isoform 1 (BRD9), Bromodomain containing testis-specific-1st bromodomain (BRDT), Bromodomain containing testis-specific-1st & 2nd bromodomains (BRDT), bromodomain testis-specific protein isoform b-bromodomain 2 (BRDT(2)), bromodomain and PHD finger containing-1 (BRPF1), bromodomain and PHD finger containing-3 (BRPF3), bromodomain and PHD finger containing-3 (BRPF3), Bromodomain and WD repeat-containing 3-2nd bromodomain (BRWD3(2)), Cat eye syndrome critical region protein 2 (CECR2), CREB binding protein (CREBBP), E1A binding protein p300 (EP300), EP300 (EP300), nucleosome-remodeling factor subunit BPTF isoform 1 (FALZ), Nucleosome-remodeling factor subunit BPT (FALZ), Euchromatic histone-lysine N-methyltransferase 2 (EHMT2), Histone Acetyltransferase-KAT2A (GCN5L2), Euchromatic histone-lysine N-methyltransferase 1 (EHMT1), Histone-lysine N-methyltransferase MLL (MLL), Polybromo 1-1st bromodomain (PB1(1)), Polybromo 1-2nd bromodomain (PB1(2)), polybromo 1-bromodomain 2 (PBRM1(2)), polybromo 1-bromodomain 5 (PBRM1(5)), Histone acetyltransferase KAT2B (PCAF), PH-interacting protein-1st bromodomain (PHIP(1)), PH-interacting protein-2nd bromodomain (PHIP(2)), Protein kinase C-binding protein 1 (PRKCBP1), Protein arginine N-methyltransferase 3 (PRMT3), SWI/SNF related-matrix associated-actin dependent regulator of chromatin-subfamily a-member 2 (SMARCA2), SWI/SNF related-matrix associated-actin dependent regulator of chromatin-subfamily a-member 4 (SMARCA4), Nuclear body protein-SP110 (SP110), Nuclear body protein-SP140 (SP140), Transcription initiation factor TFIID subunit 1 (TAF1(1-2)), TAF1 RNA polymerase II-TATA box binding protein (TBP)-associated factor-250 kDa-bromodomain 2 (TAF1(2)), Transcription initiation factor TFIID subunit 1-like-1st bromodomain (TAF1L(1)), Transcription initiation factor TFIID subunit 1-like-2nd bromodomain (TAF1L(2)), tripartite motif containing 24 (TRIM24(Bromo.)), tripartite motif containing 24 (TRIM24(PHD-Bromo.)), E3 ubiquitin-protein ligase TRIM33 (TRIM33), tripartite motif containing 33 (TRIM33(PHD-Bromo.)), WD repeat 9-1st bromodomain (WDR9(1)), and WD repeat 9-2nd bromodomain (WDR9(2)).

Examples of growth factors include, but are not limited to: nerve growth factor (NGF), vascular endothelial growth factor (VEGF), platelet-derived growth factor (PDGF), C-fos-induced growth factor (FIGF), platelet-activating factor (PAF), transforming growth factor beta (TGF-β), bone morphogenetic proteins (BMPs), Activin, inhibin, fibroblast growth factors (FGFs), granulocyte-colony stimulating factor (G-CSF), granulocyte-macrophage colony stimulating factor (GM-CSF), glial cell line-derived neurotrophic factor (GDNF), growth differentiation factor-9 (GDF9), epidermal growth factor (EGF), transforming growth factor-α (TGF-α), growth factor (KGF), migration-stimulating factor (MSF), hepatocyte growth factor-like protein (HGFLP), hepatocyte growth factor (HGF), hepatoma-derived growth factor (HDGF), and Insulin-like growth factors.

Examples of Hormones include, but are not limited to: Amino acid derived (such as melatonin and thyroxine), Thyrotropin-releasing hormone, Vasopressin, Insulin, Growth Hormones, Glycoprotein Hormones, Luteinizing Hormone, Follicle-stimulating Hormone, Thyroid-stimulating hormone, Eicosanoids, Arachidonic acid, Lipoxins, Prostaglandins, Steroid, Estrogens, Testosterone, Cortisol, and Progestogens.

Examples of Proteins and Peptides and Signal Transduction molecules include, but are not limited to: Ataxia Telangiectasia Mutated, Tumor Protein p53, Checkpoint kinase 2, breast cancer susceptibility protein, Double-strand break repair protein, DNA repair protein RAD50, Nibrin, p53-binding protein, Mediator of DNA damage checkpoint protein, H2A histone family member X, Microcephalin, C-terminal-binding protein 1, Structural maintenance of chromosomes protein 1A, Cell division cycle 25 homolog A (CDC25A), forkhead box O3 (forkhead box O3), nuclear factor of kappa light polypeptide gene enhancer in B-cells inhibitor, alpha (NFKBIA), nuclear factor (erythroid-derived 2)-like 2 (NFE2L2), Natriuretic peptide receptor A (NPR1), Tumor necrosis factor receptor superfamily, member 11a (TNFRSF11A), v-rel reticuloendotheliosis viral oncogene homolog A (avian) (RELA), Sterol regulatory element binding transcription factor 2 (SREBF2), CREB regulated transcription coactivator 1 (CRTC1), CREB regulated transcription coactivator 2 (CRTC2), X-box binding protein 1 (XBP1), Catenin beta 1 (cadherin-associated protein or CTNNB1).

Another example is Dominant Negative Tumor Necrosis Factor (DN-TNF) such as XPRO^{®}1595 and the like.

Examples of G Protein-Coupled Receptors (GPCR) include, but are not limited to: Adenosine receptor family, Adrenergic receptor family, Angiotensin II receptor, Apelin receptor, Vasopressin receptor family, Brain-specific angiogenesis inhibitor family, Bradykinin receptor family, Bombesin receptor family, Complement component 3a receptor 1, Complement component 5a receptor 1, Calcitonin receptor family, Calcitonin receptor-like family, Calcium-sensing receptor, Cholecystokinin A receptor (CCK1), Cholecystokinin B receptor (CCK2), Chemokine (C-C motif) receptor family, Sphingosine 1-phosphate receptor family, Succinic receptor, Cholinergic receptor family. Chemokine-like receptor family, Cannabinoid receptor family, Corticotropin releasing hormone receptor family, prostaglandin D2 receptor, Chemokine C-X3-C receptor family, Chemokine (C-X-C motif) receptor family, Burkitt lymphoma receptor, Chemokine (C-X-C motif) receptor family, Cysteinyl leukotriene receptor 2 (CYSLT2), chemokine receptor (FY), Dopamine receptor family, G protein-coupled receptor 183 (GPR183), Lysophosphatidic acid receptor family, Endothelin receptor family, Coagulation factor II (thrombin) receptor family, Free fatty acid receptor family, Formylpeptide receptor family, Follicle stimulating hormone receptor (FSHR), gamma-aminobutyric acid (GABA) B receptor, Galanin receptor family, Glucagon receptor, Growth hormone releasing hormone receptor (GHRH), Ghrelin receptor (ghrelin), Growth hormone secretagogue receptor 1b (GHSR1b), Gastric inhibitory polypeptide receptor (GIP), Glucagon-like peptide receptor family, Gonadotropin-releasing hormone receptor (GnRH), pyroglutamylated RFamide peptide receptor (QRFPR), G protein-coupled bile acid receptor 1 (GPBA), Hydroxycarboxylic acid receptor family, Lysophosphatidic acid receptor 4 (LPA4) Lysophosphatidic acid receptor 5 (GPR92), G protein-coupled receptor 79 pseudogene (GPR79), Hydroxycarboxylic acid receptor 1 (HCA1), G-protein coupled receptor (C5L2, FFA4, FFA4, FFA4, GPER, GPR1, GPR101, GPR107, GPR119, GPR12, GPR123, GPR132, GPR135, GPR139, GPR141, GPR142, GPR143, GPR146, GPR148, GPR149, GPR15, GPR150, GPR151, GPR152, GPR157, GPR161, GPR162, GPR17, GPR171, GPR173, GPR176, GPR18, GPR182, GPR20, GPR22, GPR25, GPR26, GPR27, GPR3, GPR31, GPR32, GPR35, GPR37L1, GPR39, GPR4, GPR45, GPR50, GPR52, GPR55, GPR6, GPR61, GPR65, GPR75, GPR78, GPR83, GPR84, GPR85, GPR88, GPR97, TM7SF1), Metabotropic glutamate receptor family, Gastrin releasing peptide receptor (BB2), Orexin receptor family, Histamine receptor family, 5-hydroxytryptamine receptor family, KISS1-derived peptide receptor (kisspeptin), Leucine-rich repeat-containing G protein-coupled receptor family, horiogonadotropin receptor (LH), Leukotriene B4 receptor (BLT1), Adenylate Cyclase Activating Polypeptide 1 Receptor 1 (mPAC1), Motilin receptor, Melanocortin receptor family, Melanin concentrating hormone receptor 1 (MCH1), Neuropeptide Y1 receptor (Y1), Neuropeptide Y2 receptor (NPY2R), Opioid receptor family, Oxytocin recepter (OT), P2Y Purinoceptor 12 (mP2Y12), P2Y Purinoceptor 6 (P2Y6), Pancreatic polypeptide receptor family, Platelet-activating factor receptor family, Prostaglandin E receptor family, Prostanoid IP1 receptor (IP1), MAS-related GPR, member family, Rhodopsin (Rhodopsin), Relaxin family peptide receptor family, Somatostatin receptor family, Tachykinin receptor family, Melatonin receptor family, Urotensin receptor family, Vasoactive intestinal peptide receptor 1 (mVPAC1), Neuromedin B Receptor (BB1), Neuromedin U receptor 1 (NMU1), Neuropeptides B/W receptor family, Neuropeptide FF receptor 1 (NPFF1), neuropeptide S receptor 1 (NPS receptor), Neuropeptide Y receptor family, Neurotensin receptor 1 (NTS1), Opsin 5 (OPN5), Opioid receptor-like receptor (NOP), Oxoeicosanoid (OXE) receptor 1 (OXE), Oxoglutarate (alpha-ketoglutarate) receptor 1 (OXGR1), Purinergic receptor family, Pyrimidinergic receptor family, Prolactin releasing hormone receptor (PRRP), Prokineticin receptor family, Platelet activating receptor (PAF), Prostaglandin F receptor family, Prostaglandin 12 (prostacyclin) receptor family, Parathyroid hormone receptor family, muscarinic acetylcholine receptors (such as rM4), Prostanoid DP2 receptor (rGPR44), Prokineticin receptor family, Relaxin family peptide receptor family, Secretin receptor (secretin), Frizzled class receptor (Smoothened), trace amine associated receptor family, Tachykinin family, Thromboxane A2 receptor (TP), Thyrotropin-releasing hormone receptor (TRH1), and Thyroid Stimulating Hormone Receptor (TSH).

Examples of nuclear hormone receptors include, but are not limited to: Androgen receptor (AR), Estrogen related receptor alpha (ESRRA), Estrogen receptor 1 (ESR1), Nuclear receptor subfamily 1-group H-member 4 (NR1H4), Nuclear receptor subfamily 3-group C-member 1 (glucocorticoid receptor) (NR3C1), Nuclear receptor subfamily 1-group H-member 3 (Liver X receptor α) (NR1H3), Nuclear receptor subfamily 1-group H-member 2 (Liver X receptor β) (NR1H2), Nuclear receptor subfamily 1-group H-member 2 (Liver X receptor β) (NR1H2), Nuclear receptor subfamily 3-group C-member 2 (Mineral corticoid receptor) (NR3C2), Peroxisome Proliferator Activated Receptor alpha (PPARA), Peroxisome Proliferator Activated Receptor gamma (PPARG), Peroxisome Proliferator Activated Receptor delta (PPARD), Progesterone receptor α (PGR), Progesterone receptor β (PGR), Retinoic acid receptor -alpha (RARA), Retinoic acid receptor -beta (RARB), Retinoid X receptor -alpha (RXRA), Retinoid X receptor -gamma (RXRG), Thyroid hormone receptor -alpha (THRA), Thyroid hormone receptor -beta (THRB), Retinoic acid-related orphan receptor, Liver X receptor, Farnesoid X receptor, Vitamin D receptor, Pregnane X receptor, Constitutive androstane receptor, Hepatocyte nuclear factor 4, Oestrogen receptor, Oestrogen-related receptor, Glucocortioic receptor, and Nerve growth factor-induced-B, Germ cell nuclear factor.

Examples of membrane transport proteins include, but are not limited to: ATP-binding cassette (ABC) superfamily, solute carrier (SLC) superfamily, multidrug resistance protein 1 (P-glycoprotein), organic anion transporter 1, and and proteins such as EAAT3, EAAC1, EAAT1, GLUT1, GLUT2, GLUT9, GLUT10, rBAT, AE1, NBC1, KNBC, CHED2, BTR1, NABC1, CDPD, SGLT1, SGLT2, NIS, CHT1, NET, DAT, GLYT2, CRTR, BOAT1, SIT1, XT3, y+LAT1, BAT1, NHERF1, NHE6, ASBT, DMT1, DCT1, NRAMP2, NKCC2, NCC, KCC3, NACT, MCT1, MCT8, MCT12, SLD, VGLUT3, THTR1, THTR2, PIT2, GLVR2, OCTN2, URAT1, NCKX1, NCKX5, CIC, PiC, ANTI, ORNT1, AGC1, ARALAR, Citrin, STLN2, aralar2, TPC, MUP1, MCPHA, CACT, GC1, PHC, DTD, CLD, DRA, PDS, Prestin, TAT1, FATP4, ENT3, ZnT2, ZnT10, AT1, NPT2A, NPT2B, HHRH, CST, CDG2F, UGAT, UGTL, UGALT, UGT1, UGT2, FUCT1, CDG2C, NST, PAT2, G6PT1, SPX4, ZIP4, LIV4, ZIP13, LZT-Hs9, FPN1, MTP1, IREG1, RHAG, AIM1, PCFT, FLVCR1, FLVCR2, RFT1, RFT2, RFT3, OATP1B1, OATP1B3, and OATP2A1.

Examples of structural proteins include, but are not limited to: tubulin, heat shock protein, Microtubule-stabilizing proteins, Oncoprotein 18, stathmin, kinesin-8 and kinesin-14 family, Kip3, and Kif18A.

Examples of proteases include, but are not limited to, ADAM (a disintegrin and metalloprotease) family.

Examples of Protein kinases include, but are not limited to: AP2 associated kinase, Homo sapiens ABL proto-oncogene 1-non-receptor tyrosine-protein kinase family, c-abl oncogene 1 receptor tyrosine kinase family, v-abl Abelson murine leukemia viral oncogene homolog 2, activin A receptor family, chaperone -ABC1 activity of bc1 complex homolog (S. pombe) (ADCK3), aarF domain containing kinase 4 (ADCK4), v-akt murine thymoma viral oncogene homolog family, anaplastic lymphoma receptor tyrosine kinase family, protein kinase A family, protein kinase B family, ankyrin repeat and kinase domain containing 1 (ANKK1), NUAK family -SNF1-like kinase, mitogen-activated protein kinase kinase kinase family aurora kinase A (AURKA), aurora kinase B (AURKB), aurora kinase C (AURKC), AXL receptor tyrosine kinase (AXL), BMP2 inducible kinase (BIKE), B lymphoid tyrosine kinase (BLK), bone morphogenetic protein receptor family, BMX non-receptor tyrosine kinase (BMX), v-raf murine sarcoma viral oncogene homolog B1 (BRAF), protein tyrosine kinase 6 (BRK), BR serine/threonine kinase family, Bruton agammaglobulinemia tyrosine kinase (BTK), calcium/calmodulin-dependent protein kinase family, cyclin-dependent kinase family, cyclin-dependent kinase-like family, CHK1 checkpoint homolog (S. pombe) (CHEK1), CHK2 checkpoint homolog (S. pombe) (CHEK2), Insulin receptor, isoform A (INSR), Insulin receptor, isoform B (INSR), rho-interacting serine/threonine kinase (CIT), v-kit Hardy-Zuckerman 4 feline sarcoma viral oncogene homolog (KIT), CDC-Like Kinase family-Hepatocyte growth factor receptor (MET), Proto-oncogene tyrosine-protein kinase receptor, colony-stimulating factor family receptor, c-src tyrosine kinase (CSK), casein kinase family, megakaryocyte-associated tyrosine kinase (CTK), death-associated protein kinase family, doublecortin-like kinase family, discoidin domain receptor tyrosine kinase, dystrophia myotonica-protein kinase (DMPK), dual-specificity tyrosine-(Y)-phosphorylation regulated kinase family, epidermal growth factor receptor family, eukaryotic translation initiation factor 2-alpha kinase 1 (EIF2AK1), EPH receptor family, Ephrin type-A receptor family, Ephrin type-B receptor family, v-erb-b2 erythroblastic leukemia viral oncogene homolog family, mitogen-activated protein kinase family, endoplasmic reticulum to nucleus signaling 1 (ERN1), PTK2 protein tyrosine kinase 2 (FAK), fer (fps/fes related) tyrosine kinase (FER). feline sarcoma oncogene (FES), Fibroblast growth factor receptor family, Gardner-Rasheed feline sarcoma viral (v-fgr) oncogene homolog (FGR), fms-related tyrosine kinase family, Fms-related tyrosine kinase family, fyn-related kinase (FRK), FYN oncogene related to SRC, cyclin G associated kinase (GAK), eukaryotic translation initiation factor 2 alpha kinase, Growth hormone receptor. G protein-coupled receptor kinase 1 (GRK1), G protein-coupled receptor kinase family, glycogen synthase kinase family, germ cell associated 2 (haspin) (HASPIN), Hemopoietic cell kinase (HCK), homeodomain interacting protein kinase family, mitogen-activated protein kinase kinase kinase kinase family, hormonally up-regulated Neu-associated kinase (HUNK), intestinal cell (MAK-like) kinase (ICK), Insulin-like growth factor 1 receptor (IGF1R), conserved helix-loop-helix ubiquitous kinase (IKK-alpha), inhibitor of kappa light polypeptide gene enhancer in B-cells -kinase beta family, insulin receptor (INSR), insulin receptor-related receptor (INSRR), interleukin-1 receptor-associated kinase family, IL2-inducible T-cell kinase (ITK), Janus kinase family, Kinase Insert Domain Receptor, v-kit Hardy-Zuckerman 4 feline sarcoma viral oncogene homolog, lymphocyte-specific protein tyrosine kinase (LCK), LIM domain kinase family, serine/threonine kinase family leucine-rich repeat kinase family, v-yes-1 Yamaguchi sarcoma viral related oncogene homolog (LYN), male germ cell-associated kinase (MAK); MAP/microtubule affinity-regulating kinase family such as microtubule associated serine/threonine kinase family, maternal embryonic leucine zipper kinase, c-mer proto-oncogene tyrosine kinase (MERTK), met proto-oncogene (hepatocyte growth factor receptor), MAP kinase interacting serine/threonine kinase family, myosin light chain kinase family, mixed lineage kinase domain-like protein isoform, CDC42 binding protein kinase family, serine/threonine kinase family, macrophage stimulating 1 receptor (c-met-related tyrosine kinase) (MST1R), mechanistic target of rapamycin (serine/threonine kinase) (MTOR), muscle-skeletal-receptor tyrosine kinase (MUSK), myosin light chain kinase family, NIMA (never in mitosis gene a)-related kinase family, serine/threonine-protein kinase NIM1 (NIM1), nemo-like kinase (NLK), oxidative-stress responsive 1 (OSR1), p21 protein (Cdc42/Rac)-activated kinase family, PAS domain containing serine/threonine kinase, Platelet-derived growth factor receptor family, 3-phosphoinositide dependent protein kinase-1 (PDPK1), Calcium-dependent protein kinase 1, phosphorylase kinase gamma family, Phosphatidylinositol 4, 5-bisphosphate 3-kinase, phosphoinositide-3-kinase family, phosphatidylinositol 4-kinase family. phosphoinositide kinase, FYVE finger containing, Pim-1 oncogene (PIM1), pim-2 oncogene (PIM2), pim-3 oncogene (PIM3), phosphatidylinositol-4-phosphate 5-kinase family, phosphatidylinositol-5-phosphate 4-kinase family protein kinase, membrane associated tyrosine/threonine 1 (PKMYT1), protein kinase N family, polo-like kinase family, protein kinase C family, protein kinase D family, cGMP-dependent protein kinase family, eukaryotic translation initiation factor 2-alpha kinase 2 (PRKR), X-linked protein kinase (PRKX), Prolactin receptor (PRLR), PRP4 pre-mRNA processing factor 4 homolog B (yeast) (PRP4), PTK2B protein tyrosine kinase 2 beta (PTK2B), SIK family kinase 3 (QSK), v-raf-1 murine leukemia viral oncogene homolog 1 (RAF1), Neurotrophic tyrosine kinase receptor type family, receptor (TNFRSF)-interacting serine-threonine kinase family, dual serine/threonine and tyrosine protein kinase (RIPK5), Rho-associated, coiled-coil containing protein kinase family, c-ros oncogene 1, receptor tyrosine kinase (ROS1), ribosomal protein S6 kinase family, SH3-binding domain kinase 1 (SBK1), serum/glucocorticoid regulated kinase family, Putative uncharacterized serine/threonine-protein kinase (Sugen kinase 110) (SgK110), salt-inducible kinase family, SNF related kinase (SNRK), src-related kinase, SFRS protein kinase family; Spleen tyrosine kinase (SYK) such as TAO kinase family; TANK-binding kinase 1 (TBK1) such as tec protein tyrosine kinase (TEC), testis-specific kinase 1 (TESK1), transforming growth factor, beta receptor family, tyrosine kinase with immunoglobulin-like and EGF-like domains 1 (TIE1), TEK tyrosine kinase, endothelial (TIE2), Angiopoietin-1 receptor (Tie2), tousled-like kinase family, TRAF2 and NCK interacting kinase (TN IK), non-receptor tyrosine kinase family, TNNI3 interacting kinase (TNNI3K), transient receptor potential cation channel, testis-specific serine kinase family, TTK protein kinase (TTK), TXK tyrosine kinase (TXK), Tyrosine kinase 2 (TYK2), TYRO3 protein tyrosine kinase (TYRO3), unc-51-like kinase family, phosphatidylinositol 3-kinase, vaccinia related kinase 2 (VRK2), WEE1 homolog family, WNK lysine deficient protein kinase family, v-yes-1 Yamaguchi sarcoma viral oncogene homolog 1 (YES), sterile alpha motif and leucine zipper containing kinase AZK (ZAK), and zeta-chain (TCR) associated protein kinase 70 kDa (ZAP70).

Cell therapy using cells that are derived primarily from: endoderm such as Exocrine secretory epithelial cells and Hormone-secreting cells; ectoderm such as Keratinizing epithelial cells, Wet stratified barrier epithelial cells, Sensory transducer cells, Autonomic neuron cells, Sense organ and peripheral neuron supporting cells, Central nervous system neurons and glial cells, Lens cells; mesoderm such as Metabolism and storage cells, Barrier function cells (lung, gut, exocrine glands and urogenital tract), Extracellular matrix cells, Contractile cells, Blood and immune system cells, Germ cells, Nurse cell, Interstitial cells and combinations thereof.

Examples of Exocrine secretory epithelial cells include but are not limited to: Salivary gland mucous cell, Salivary gland number 1, Von Ebner's gland cell in tongue, Mammary gland cell, Lacrimal gland cell, Ceruminous gland cell in ear, Eccrine sweat gland dark cell, Eccrine sweat gland clear cell, Apocrine sweat gland cell, Gland of Moll cell in eyelid, Sebaceous gland cell, Bowman's gland cell in nose, Brunner's gland cell in duodenum, Seminal vesicle cell, Prostate gland cell, Bulbourethral gland cell, Bartholin's gland cell, Gland of Littre cell, Uterus endometrium cell, Isolated goblet cell of respiratory and digestive tracts, Stomach lining mucous cell, Gastric gland zymogenic cell, Gastric gland oxyntic cell, Pancreatic acinar cell, Paneth cell of small intestine, Type II pneumocyte of lung, and Clara cell of lung; Hormone-secreting cells including, but not limited to: Anterior pituitary cells, Intermediate pituitary cell, Magnocellular neurosecretory cells, Gut and respiratory tract cells, Thyroid gland cells, Parathyroid gland cells, Adrenal gland cells, Leydig cell of testes secreting testosterone, Theca interna cell of ovarian follicle secreting estrogen, Corpus luteum cell of ruptured ovarian follicle secreting progesterone, Juxtaglomerular cell, Macula densa cell of kidney, Peripolar cell of kidney, Mesangial cell of kidney, and Pancreatic islets; Keratinizing epithelial cells including, but not limited to: Epidermal keratinocyte, Epidermal basal cell, Keratinocyte of fingernails and toenails, Nail bed basal cell, Medullary hair shaft cell, Cortical hair shaft cell, Cuticular hair shaft cell, Cuticular hair root sheath cell, Hair root sheath cell of Huxley's layer, Hair root sheath cell of Henle's layer, External hair root sheath cell, and Hair matrix cell; Wet stratified barrier epithelial cells including, but not limited to: Surface epithelial cell of stratified squamous epithelium and basal cell of epithelia of cornea, tongue, oral cavity, esophagus, anal canal, distal urethra and vagina, and Urinary epithelium cell; Sensory transducer cells including, but not limited to: Auditory inner hair cell of organ of Corti, Auditory outer hair cell of organ of Corti, Basal cell of olfactory epithelium, Cold-sensitive primary sensory neurons, Heat-sensitive primary sensory neurons, Merkel cell of epidermis, Olfactory receptor neuron, Pain-sensitive primary sensory neurons, Photoreceptor cells of retina in eye, Proprioceptive primary sensory neurons, Touch-sensitive primary sensory neurons, Type I carotid body cell, Type II carotid body cell, Type I hair cell of vestibular system of ear, Type II hair cell of vestibular system of ear, and Type I taste bud cell; Autonomic neuron cells including, but not limited to: Cholinergic neural cell, Adrenergic neural cell, and Peptidergic neural cell; Sense organ and peripheral neuron supporting cells including, but not limited to: Inner pillar cell of organ of Corti, Outer pillar cell of organ of Corti, Inner phalangeal cell of organ of Corti, Outer phalangeal cell of organ of Corti, Border cell of organ of Corti, Hensen cell of organ of Corti, Vestibular apparatus supporting cell, Taste bud supporting cell, Olfactory epithelium supporting cell, Schwann cell, Satellite glial cell, and Enteric glial cell; Central nervous system neurons and glial cells including, but not limited to: Astrocyte, Neuron cells, Oligodendrocyte, and Spindle neuron; Lens cells including, but not limited to: Anterior lens epithelial cell, and Crystallin-containing lens fiber cell; Metabolism and storage cells including, but not limited to: Adipocytes, and Liver lipocyte; Barrier function cells including, but not limited to: Kidney parietal cell, Kidney glomerulus podocyte, Kidney proximal tubule brush border cell, Loop of Henle thin segment cell, Kidney distal tubule cell, Kidney collecting duct cell, Principal cells, Intercalated cells, Type I pneumocyte, Pancreatic duct cell, Nonstriated duct cell, Principal cell, Intercalated cell, Duct cell, Intestinal brush border cell, Exocrine gland striated duct cell, Gall bladder epithelial cell, Ductulus efferens nonciliated cell, Epididymal principal cell, and Epididymal basal cell; Extracellular matrix cells including, but not limited to: Ameloblast epithelial cell, Planum semilunatum epithelial cell of vestibular system of ear, Organ of Corti interdental epithelial cell, Loose connective tissue fibroblasts, Corneal fibroblasts, Tendon fibroblasts, Bone marrow reticular tissue fibroblasts, Other nonepithelial fibroblasts, Pericyte, Nucleus pulposus cell of intervertebral disc, Cementoblast/cementocyte, Odontoblast/odontocyte, Hyaline cartilage chondrocyte, Fibrocartilage chondrocyte, Elastic cartilage chondrocyte, Osteoblast/osteocyte, Osteoprogenitor cell, Hyalocyte of vitreous body of eye, Stellate cell of perilymphatic space of ear, Hepatic stellate cell, and Pancreatic stelle cell; Contractile cells including, but not limited to: Skeletal muscle cell, Satellite cell, Heart muscle cells, Smooth muscle cell, Myoepithelial cell of iris, and Myoepithelial cell of exocrine glands; Blood and immune system cells including, but not limited to: Erythrocyte, Megakaryocyte, Monocyte, Connective tissue macrophage, Epidermal Langerhans cell, Osteoclast, Dendritic cell, Microglial cell, Neutrophil granulocyte, Eosinophil granulocyte, Basophil granulocyte, Hybridoma cell, Mast cell, Helper T cell, Suppressor T cell, Cytotoxic T cell, Natural Killer T cell, B cell, Natural killer cell, Reticulocyte, Stem cells, and committed progenitors for the blood and immune system; Germ cells including, but not limited to: Oogonium/Oocyte, Spermatid, Spermatocyte, Spermatogonium cell, and Spermatozoon; Nurse cell including, but not limited to: Ovarian follicle cell, and Sertoli cell, Thymus epithelial cell; Interstitial cells including, but not limited to: Interstitial kidney cells and any combination of the foregoing.

Non-limiting examples of other known biologics include, but are not limited to: Abbosynagis, Abegrin, Actemra, AFP-Cide, Antova, Arzerra, Aurexis, Avastin, Benlysta, Bexxar, Blontress, Bosatria, Campath, CEA-Cide, CEA-Scan, Cimzia, Cyramza, Ektomab, Erbitux, FibriScint, Gazyva, Herceptin, hPAM4-Cide, HumaSPECT, HuMax-CD4, HuMax-EGFr, Humira, HuZAF, Hybri-ceaker, Ilaris, Indimacis-125, Kadcyla, Lemtrada, LeukArrest, LeukoScan, Lucentis, Lymphomun, LymphoScan, LymphoStat-B, MabThera, Mycograb, Mylotarg, Myoscint, NeutroSpec, Numax, Nuvion, Omnitarg, Opdivo, Orthoclone OKT3, OvaRex, Panorex, Prolia, Prostascint, Raptiva, Remicade, Removab, Rencarex, ReoPro, Rexomun, Rituxan, RoActemra, Scintimun, Simponi, Simulect, Soliris, Stelara, Synagis, Tactress, Theracim, Theragyn, Theraloc, Tysabri, Vectibix, Verluma, Xolair, Yervoy, Zenapax, and Zevalin and combinations thereof.

Non-limiting examples of known Monoclonal antibodies include, but are not limited to: 3F8, 8H9, Abagovomab, Abciximab, Abituzumab, Abrilumab, Actoxumab, Adalimumab, Adecatumumab, Aducanumab, Afasevikumab, Afelimomab, Afutuzumab, Alacizumab pegol, ALD518, ALD403, Alemtuzumab, Alirocumab, Altumomab pentetate, Amatuximab, AMG 334, Anatumomab mafenatox, Anetumab ravtansine, Anifrolumab, Anrukinzumab, Apolizumab, Arcitumomab, Ascrinvacumab, Aselizumab, Atezolizumab, Atinumab, Atlizumab, Atorolimumab, Avelumab, Bapineuzumab, Basiliximab, Bavituximab, Bectumomab, Begelomab, Belimumab, Benralizumab, Bertilimumab, Besilesomab, Bevacizumab, Bezlotoxumab, Biciromab, Bimagrumab, Bimekizumab, Bivatuzumab mertansine, Bleselumab, Blinatumomab, Blontuvetmab, Blosozumab, Bococizumab, Brazikumab, Brentuximab vedotin, Briakinumab, Brodalumab, Brolucizumab, Brontictuzumab, Burosumab, Cabiralizumab, Canakinumab, Cantuzumab mertansine, Cantuzumab ravtansine, Caplacizumab, Capromab pendetide, Carlumab, Carotuximab, Catumaxomab, cBR96-doxorubicin immunoconjugate, Cedelizumab, Cergutuzumab amunaleukin, Certolizumab pegol, Cetuximab, Citatuzumab bogatox, Cixutumumab, Clazakizumab, Clenoliximab, Clivatuzumab tetraxetan, Codrituzumab, Coltuximab ravtansine, Conatumumab, Concizumab, CR6261, Crenezumab, Crotedumab, Dacetuzumab, Daclizumab, Dalotuzumab, Dapirolizumab pegol, Daratumumab, Dectrekumab, Demcizumab, Denintuzumab mafodotin, Denosumab, Depatuxizumab mafodotin, Derlotuximab biotin, Detumomab, Dinutuximab, Diridavumab, Domagrozumab, Dorlimomab aritox, Drozitumab, Duligotumab, Dupilumab, Durvalumab, Dusigitumab, Ecromeximab, Eculizumab, Edobacomab, Edrecolomab, Efalizumab, Efungumab, Eldelumab, Elgemtumab, Elotuzumab, Elsilimomab, Emactuzumab, Emibetuzumab, Emicizumab, Enavatuzumab, Enfortumab vedotin, Enlimomab pegol, Enoblituzumab, Enokizumab, Enoticumab, Ensituximab, Epitumomab cituxetan, Epratuzumab, Erenumab, Erlizumab, Ertumaxomab, Etaracizumab, Etrolizumab, Evinacumab, Evolocumab, Exbivirumab, Fanolesomab, Faralimomab, Farletuzumab, Fasinumab, FBTA05, Felvizumab, Fezakinumab, Fibatuzumab, Ficlatuzumab, Figitumumab, Firivumab, Flanvotumab, Fletikumab, Fontolizumab, Foralumab, Foravirumab, Fresolimumab, Fulranumab, Futuximab, Galcanezumab, Galiximab, Ganitumab, Gantenerumab, Gavilimomab, Gemtuzumab ozogamicin, Gevokizumab, Girentuximab, Glembatumumab vedotin, Golimumab, Gomiliximab, Guselkumab, Ibalizumab, Ibritumomab tiuxetan, Icrucumab, Idarucizumab, Igovomab, IMA-638, IMAB362, Imalumab, Imciromab, Imgatuzumab, Inclacumab, Indatuximab ravtansine, Indusatumab vedotin, Inebilizumab, Infliximab, Inolimomab, Inotuzumab ozogamicin, Intetumumab, Ipilimumab, Iratumumab, Isatuximab, Itolizumab, Ixekizumab, Keliximab, Labetuzumab, Lambrolizumab, Lampalizumab, Lanadelumab, Landogrozumab, Laprituximab emtansine, LBR-101/PF0442g7429, Lebrikizumab, Lemalesomab, Lendalizumab, Lenzilumab, Lerdelimumab, Lexatumumab, Libivirumab, Lifastuzumab vedotin, Ligelizumab, Lilotomab satetraxetan, Lintuzumab, Lirilumab, Lodelcizumab, Lokivetmab, Lorvotuzumab mertansine, Lucatumumab, Lulizumab pegol, Lumiliximab, Lumretuzumab, LY2951742, Mapatumumab, Margetuximab, Maslimomab, Matuzumab, Mavrilimumab, Mepolizumab, Metelimumab, Milatuzumab, Minretumomab, Mirvetuximab soravtansine, Mitumomab, Mogamulizumab, Monalizumab, Morolimumab, Motavizumab, Moxetumomab pasudotox, Muromonab-CD3, Nacolomab tafenatox, Nam ilumab, Naptumomab estafenatox, Naratuximab emtansine, Narnatumab, Natalizumab, Navicixizumab, Navivumab, Nebacumab, Necitumumab, Nemolizumab, Nerelimomab, Nesvacumab, Nimotuzumab, Nivolumab, Nofetumomab merpentan, Obiltoxaximab, Obinutuzumab, Ocaratuzumab, Ocrelizumab, Odulimomab, Ofatumumab, Olaratumab, Olokizumab, Omalizumab, Onartuzumab, Ontuxizumab, Opicinumab, Oportuzumab monatox, Oregovomab, Orticumab, Otelixizumab, Otlertuzumab, Oxelumab, Ozanezumab, Ozoralizumab, Pagibaximab, Palivizumab, Pamrevlumab, Panitumumab, Pankomab, Panobacumab, Parsatuzumab, Pascolizumab, Pasotuxizumab, Pateclizumab, Patritumab, Pembrolizumab, Pemtumomab, Perakizumab, Pertuzumab, Pexelizumab, Pidilizumab, Pinatuzumab vedotin, Pintumomab, Placulumab, Plozalizumab, Pogalizumab, Polatuzumab vedotin, Ponezumab, Prezalizumab, Priliximab, Pritoxaximab, Pritumumab, PRO 140, Quilizumab, Racotumomab, Radretumab, Rafivirumab, Ralpancizumab, Ramucirumab, Ranibizumab, Raxibacumab, Refanezumab, Regavirumab, Reslizumab, Rilotumumab, Rinucumab, Risankizumab, Rituximab, Rivabazumab pegol, Robatumumab, Roledumab, Romosozumab, Rontalizumab, Rovalpituzumab tesirine, Rovelizumab, Ruplizumab, Sacituzumab govitecan, Samalizumab, Sapelizumab, Sarilumab, Satumomab pendetide, Secukinumab, Seribantumab, Setoxaximab, Sevirumab, SGN-CD19A, SGN-CD33A, Sibrotuzumab, Sifalimumab, Siltuximab, Simtuzumab, Siplizumab, Sirukumab, Sofituzumab vedotin, Solanezumab, Solitomab, Sonepcizumab, Sontuzumab, Stamulumab, Sulesomab, Suvizumab, Tabalumab, Tacatuzumab tetraxetan, Tadocizumab, Talizumab, Tamtuvetmab, Tanezumab, Taplitumomab paptox, Tarextumab, Tefibazumab, Telimomab aritox, Tenatumomab, Teneliximab, Teplizumab, Teprotumumab, Tesidolumab, Tetulomab, Tezepelumab, TGN1412, Ticilimumab, Tigatuzumab, Tildrakizumab, Timolumab, Tisotumab vedotin, TNX-650, Tocilizumab, Toralizumab, Tosatoxumab, Tositumomab, Tovetumab, Tralokinumab, Trastuzumab, Trastuzumab emtansine, TRBS07, Tregalizumab, Tremelimumab, Trevogrumab, Tucotuzumab celmoleukin, Tuvirumab, Ublituximab, Ulocuplumab, Urelumab, Urtoxazumab, Ustekinumab, Utomilumab, Vadastuximab talirine, Vandortuzumab vedotin, Vantictumab, Vanucizumab, Vapaliximab, Varlilumab, Vatelizumab, Vedolizumab, Veltuzumab, Vepalimomab, Vesencumab, Visilizumab, Vobarilizumab, Volociximab, Vorsetuzumab mafodotin, Votumumab, Xentuzumab, Zalutumumab, Zanolimumab, Zatuximab, Ziralimumab, and Zolimomab aritox and combinations thereof.

Examples of vaccines developed for viral diseases include, but are not limited to: Hepatitis A vaccine, Hepatitis B vaccine, Hepatitis E vaccine, HPV vaccine, Influenza vaccine, Japanese encephalitis vaccine, MMR vaccine, MMRV vaccine, Polio vaccine, Rabies vaccine, Rotavirus vaccine, Varicella vaccine, Shingles vaccine, Smallpox vaccine, Yellow Fever vaccine, Adenovirus vaccine, Coxsackie B virus vaccine, Cytomegalovirus vaccine, Dengue vaccine for humans, Eastern Equine encephalitis virus vaccine for humans, Ebola vaccine, Enterovirus 71 vaccine, Epstein-Barr vaccine, Hepatitis C vaccine, HIV vaccine, HTLV-1 T-lymphotropic leukemia vaccine for humans, Marburg virus disease vaccine, Norovirus vaccine, Respiratory syncytial virus vaccine for humans, Severe acute respiratory syndrome (SARS) vaccine, West Nile virus vaccine for humans; Examples of bacterial diseases include but are not limited to: Anthrax vaccines, DPT vaccine, Q fever vaccine, Hib vaccine, Tuberculosis (BCG) vaccine, Meningococcal vaccine, Typhoid vaccine, Pneumococcal conjugate vaccine, Pneumococcal polysaccharide vaccine, Cholera vaccine, Caries vaccine, Ehrlichiosis vaccine, Leprosy vaccine, Lyme disease vaccine, Staphylococcus aureus vaccine, Streptococcus pyogenes vaccine, Syphilis vaccine, Tularemia vaccine, and Yersinia pestis vaccine; Examples of parasitic diseases include, but are not limited to: Malaria vaccine, Schistosomiasis vaccine, Chagas disease vaccine, Hookworm vaccine, Onchocerciasis river blindness vaccine for humans, Trypanosomiasis vaccine, and Visceral leishmaniasis vaccine; Examples of non-infectious diseases include, but are not limited to: Alzheimer's disease amyloid protein vaccine, Breast cancer vaccine, Ovarian cancer vaccine, Prostate cancer vaccine, and Talimogene laherparepvec (T-VEC); also vaccines including, but not limited to the following trade names: ACAM2000, ActHIB, Adacel, Afluria, AFLURIA QUADRIVALENT, Agriflu, BCG Vaccine, BEXSERO, Biothrax, Boostrix, Cervarix, Comvax, DAPTACEL, DECAVAC, Engerix-B, FLUAD, Fluarix, Fluarix Quadrivalent, Flublok, Flucelvax, Flucelvax Quadrivalent, FluLaval, FluMist, FluMist Quadrivalent, Fluvirin, Fluzone Quadrivalent, Fluzone, Fluzone High-Dose and Fluzone Intradermal, Gardasil, Gardasil 9, Havrix, Hiberix, Imovax, Infanrix, IPOL, Ixiaro, JE-Vax, KINRIX, Menactra, MenHibrix, Menomune-A/C/Y/W-135, Menveo, M-M-R II, M-M-Vax, Pediarix, PedvaxHIB, Pentacel, Pneumovax 23, Poliovax, Prevnar, Prevnar 13, ProQuad, Quadracel, Quadrivalent, RabAvert, Recombivax HB, ROTARIX, RotaTeq, TENIVAC, TICE BCG, Tripedia, TRUMENBA, Twinrix, TYPHIM Vi, VAQTA, Varivax, Vaxchora, Vivotif, YF-Vax, Zostavax, and combinations thereof.

Examples of injectable drugs include, but are not limited to: Ablavar (Gadofosveset Trisodium Injection), Abarelix Depot, Abobotulinumtoxin A Injection (Dysport), ABT-263, ABT-869, ABX-EFG, Accretropin (Somatropin Injection), Acetadote (Acetylcysteine Injection), Acetazolamide Injection (Acetazolamide Injection), Acetylcysteine Injection (Acetadote), Actemra (Tocilizumab Injection), Acthrel (Corticorelin Ovine Triflutate for Injection), Actummune, Activase, Acyclovir for Injection (Zovirax Injection), Adacel, Adalimumab, Adenoscan (Adenosine Injection), Adenosine Injection (Adenoscan), Adrenaclick, AdreView (Iobenguane 1123 Injection for Intravenous Use), Afluria, Ak-Fluor (Fluorescein Injection), Aldurazyme (Laronidase), Alglucerase Injection (Ceredase), Alkeran Injection (Melphalan Hcl Injection), Allopurinol Sodium for Injection (Aloprim), Aloprim (Allopurinol Sodium for Injection), Alprostadil, Alsuma (Sumatriptan Injection), ALTU-238, Amino Acid Injections, Aminosyn, Apidra, Apremilast, Alprostadil Dual Chamber System for Injection (Caverject Impulse), AMG 009, AMG 076, AMG 102, AMG 108, AMG 114, AMG 162, AMG 220, AMG 221, AMG 222, AMG 223, AMG 317, AMG 379, AMG 386, AMG 403, AMG 477, AMG 479, AMG 517, AMG 531, AMG 557, AMG 623, AMG655, AMG 706, AMG 714, AMG 745, AMG 785, AMG 811, AMG 827, AMG 837, AMG 853, AMG 951, Amiodarone HCl Injection (Amiodarone HCl Injection), Amobarbital Sodium Injection (Amytal Sodium), Amytal Sodium (Amobarbital Sodium Injection), Anakinra, Anti-Abeta, Anti-Beta7, Anti-Beta20, Anti-CD4, Anti-CD20, Anti-CD40, Anti-IFNalpha, Anti-IL13, Anti-OX40L, Anti-oxLDS, Anti-NGF, Anti-NRP1, Arixtra, Amphadase (Hyaluronidase Inj), Ammonul (Sodium Phenylacetate and Sodium Benzoate Injection), Anaprox, Anzemet Injection (Dolasetron Mesylate Injection), Apidra (Insulin Glulisine [rDNA origin] Inj), Apomab, Aranesp (darbepoetin alfa), Argatroban (Argatroban Injection), Arginine Hydrochloride Injection (R-Gene 10, Aristocort, Aristospan, Arsenic Trioxide Injection (Trisenox), Articane HCl and Epinephrine Injection (Septocaine), Arzerra (Ofatumumab Injection), Asclera (Polidocanol Injection), Ataluren, Ataluren-DMD, Atenolol Inj (Tenormin I.V. Injection), Atracurium Besylate Injection (Atracurium Besylate Injection), Avastin, Azactam Injection (Aztreonam Injection), Azithromycin (Zithromax Injection), Aztreonam Injection (Azactam Injection), Baclofen Injection (Lioresal Intrathecal), Bacteriostatic Water (Bacteriostatic Water for Injection), Baclofen Injection (Lioresal Intrathecal), Bal in Oil Ampules (Dimercarprol Injection), BayHepB, BayTet, Benadryl, Bendamustine Hydrochloride Injection (Treanda), Benztropine Mesylate Injection (Cogentin), Betamethasone Injectable Suspension (Celestone Soluspan), Bexxar, Bicillin C-R 900/300 (Penicillin G Benzathine and Penicillin G Procaine Injection), Blenoxane (Bleomycin Sulfate Injection), Bleomycin Sulfate Injection (Blenoxane), Boniva Injection (Ibandronate Sodium Injection), Botox Cosmetic (OnabotulinumtoxinA for Injection), BR3-FC, Bravelle (Urofollitropin Injection), Bretylium (Bretylium Tosylate Injection), Brevital Sodium (Methohexital Sodium for Injection), Brethine, Briobacept, BTT-1023, Bupivacaine HCl, Byetta, Ca-DTPA (Pentetate Calcium Trisodium Inj), Cabazitaxel Injection (Jevtana), Caffeine Alkaloid (Caffeine and Sodium Benzoate Injection), Calcijex Injection (Calcitrol), Calcitrol (Calcijex Injection), Calcium Chloride (Calcium Chloride Injection 10%), Calcium Disodium Versenate (Edetate Calcium Disodium Injection), Campath (Altemtuzumab), Camptosar Injection (Irinotecan Hydrochloride), Canakinumab Injection (Ilaris), Capastat Sulfate (Capreomycin for Injection), Capreomycin for Injection (Capastat Sulfate), Cardiolite (Prep kit for Technetium Tc99 Sestamibi for Injection), Carticel, Cathflo, Cefazolin and Dextrose for Injection (Cefazolin Injection), Cefepime Hydrochloride, Cefotaxime, Ceftriaxone, Cerezyme, Carnitor Injection, Caverject, Celestone Soluspan, Celsior, Cerebyx (Fosphenytoin Sodium Injection), Ceredase (Alglucerase Injection), Ceretec (Technetium Tc99m Exametazime Injection), Certolizumab, CF-101, Chloramphenicol Sodium Succinate (Chloramphenicol Sodium Succinate Injection), Chloramphenicol Sodium Succinate Injection (Chloramphenicol Sodium Succinate), Cholestagel (Colesevelam HCL), Choriogonadotropin Alfa Injection (Ovidrel), Cimzia, Cisplatin (Cisplatin Injection), Clolar (Clofarabine Injection), Clomiphine Citrate, Clonidine Injection (Duraclon), Cogentin (Benztropine Mesylate Injection), Colistimethate Injection (Coly-Mycin M), Coly-Mycin M (Colistimethate Injection), Compath, Conivaptan Hcl Injection (Vaprisol), Conjugated Estrogens for Injection (Premarin Injection), Copaxone, Corticorelin Ovine Triflutate for Injection (Acthrel), Corvert (Ibutilide Fumarate Injection), Cubicin (Daptomycin Injection), CF-101, Cyanokit (Hydroxocobalamin for Injection), Cytarabine Liposome Injection (DepoCyt), Cyanocobalamin, Cytovene (ganciclovir), D.H.E. 45, Dacetuzumab, Dacogen (Decitabine Injection), Dalteparin, Dantrium IV (Dantrolene Sodium for Injection), Dantrolene Sodium for Injection (Dantrium IV), Daptomycin Injection (Cubicin), Darbepoietin Alfa, DDAVP Injection (Desmopressin Acetate Injection), Decavax, Decitabine Injection (Dacogen), Dehydrated Alcohol (Dehydrated Alcohol Injection), Denosumab Injection (Prolia), Delatestryl, Delestrogen, Delteparin Sodium, Depacon (Valproate Sodium Injection), Depo Medrol (Methylprednisolone Acetate Injectable Suspension), DepoCyt (Cytarabine Liposome Injection), DepoDur (Morphine Sulfate XR Liposome Injection), Desmopressin Acetate Injection (DDAVP Injection), Depo-Estradiol, Depo-Provera 104 mg/ml, Depo-Provera 150 mg/ml, Depo-Testosterone, Dexrazoxane for Injection, Intravenous Infusion Only (Totect), Dextrose/Electrolytes, Dextrose and Sodium Chloride Inj (Dextrose 5% in 0.9% Sodium Chloride), Dextrose, Diazepam Injection (Diazepam Injection), Digoxin Injection (Lanoxin Injection), Dilaudid-HP (Hydromorphone Hydrochloride Injection), Dimercarprol Injection (Bal in Oil Ampules), Diphenhydramine Injection (Benadryl Injection), Dipyridamole Injection (Dipyridamole Injection), DMOAD, Docetaxel for Injection (Taxotere), Dolasetron Mesylate Injection (Anzemet Injection), Doribax (Doripenem for Injection), Doripenem for Injection (Doribax), Doxercalciferol Injection (Hectorol Injection), Doxil (Doxorubicin Hcl Liposome Injection), Doxorubicin Hcl Liposome Injection (Doxil), Duraclon (Clonidine Injection), Duramorph (Morphine Injection), Dysport (Abobotulinumtoxin A Injection), Ecallantide Injection (Kalbitor), EC-Naprosyn (naproxen), Edetate Calcium Disodium Injection (Calcium Disodium Versenate), Edex (Alprostadil for Injection), Engerix, Edrophonium Injection (Enlon), Eliglustat Tartate, Eloxatin (Oxaliplatin Injection), Emend Injection (Fosaprepitant Dimeglumine Injection), Enalaprilat Injection (Enalaprilat Injection), Enlon (Edrophonium Injection), Enoxaparin Sodium Injection (Lovenox), Eovist (Gadoxetate Disodium Injection), Enbrel (etanercept), Enoxaparin, Epicel, Epinepherine, Epipen, Epipen Jr., Epratuzumab, Erbitux, Ertapenem Injection (Invanz), Erythropoieten, Essential Amino Acid Injection (Nephramine), Estradiol Cypionate, Estradiol Valerate, Etanercept, Exenatide Injection (Byetta), Evlotra, Fabrazyme (Adalsidase beta), Famotidine Injection, FDG (Fludeoxyglucose F 18 Injection), Feraheme (Ferumoxytol Injection), Feridex I.V. (Ferumoxides Injectable Solution), Fertinex, Ferumoxides Injectable Solution (Feridex I.V.), Ferumoxytol Injection (Feraheme), Flagyl Injection (Metronidazole Injection), Fluarix, Fludara (Fludarabine Phosphate), Fludeoxyglucose F 18 Injection (FDG), Fluorescein Injection (Ak-Fluor), Follistim AQ Cartridge (Follitropin Beta Injection), Follitropin Alfa Injection (Gonal-f RFF), Follitropin Beta Injection (Follistim AQ Cartridge), Folotyn (Pralatrexate Solution for Intravenous Injection), Fondaparinux, Forteo (Teriparatide (rDNA origin) Injection), Fostamatinib, Fosaprepitant Dimeglumine Injection (Emend Injection), Foscarnet Sodium Injection (Foscavir), Foscavir (Foscarnet Sodium Injection), Fosphenytoin Sodium Injection (Cerebyx), Fospropofol Disodium Injection (Lusedra), Fragmin, Fuzeon (enfuvirtide), GA101, Gadobenate Dimeglumine Injection (Multihance), Gadofosveset Trisodium Injection (Ablavar), Gadoteridol Injection Solution (ProHance), Gadoversetamide Injection (OptiMARK), Gadoxetate Disodium Injection (Eovist), Ganirelix (Ganirelix Acetate Injection), Gardasil, GC1008, GDFD, Gemtuzumab Ozogamicin for Injection (Mylotarg), Genotropin, Gentamicin Injection, GENZ-112638, Golimumab Injection (Simponi Injection), Gonal-f RFF (Follitropin Alfa Injection), Granisetron Hydrochloride (Kytril Injection), Gentamicin Sulfate, Glatiramer Acetate, Glucagen, Glucagon, HAE1, Haldol (Haloperidol Injection), Havrix, Hectorol Injection (Doxercalciferol Injection), Hedgehog Pathway Inhibitor, Heparin, Herceptin, hG-CSF, Humalog, Human Growth Hormone, Humatrope, HuMax, Humegon, Humira, Humulin, Ibandronate Sodium Injection (Boniva Injection), Ibuprofen Lysine Injection (NeoProfen), Ibutilide Fumarate Injection (Corvert), Idamycin PFS (Idarubicin Hydrochloride Injection), Idarubicin Hydrochloride Injection (Idamycin PFS), Ilaris (Canakinumab Injection), Imipenem and Cilastatin for Injection (Primaxin I.V.), Imitrex, Incobotulinumtoxin A for Injection (Xeomin), Increlex (Mecasermin [rDNA origin] Injection), Indocin IV (Indomethacin Inj), Indomethacin Inj (Indocin IV), Infanrix, Innohep, Insulin, Insulin Aspart [rDNA origin] Inj (NovoLog), Insulin Glargine [rDNA origin] Injection (Lantus), Insulin Glulisine [rDNA origin] Inj (Apidra), Interferon alfa-2b, Recombinant for Injection (Intron A), Intron A (Interferon alfa-2b, Recombinant for Injection), Invanz (Ertapenem Injection), Invega Sustenna (Paliperidone Palmitate Extended-Release Injectable Suspension), Invirase (saquinavir mesylate), lobenguane 1123 Injection for Intravenous Use (AdreView), Iopromide Injection (Ultravist), Ioversol Injection (Optiray Injection), Iplex (Mecasermin Rinfabate [rDNA origin] Injection), Iprivask, Irinotecan Hydrochloride (Camptosar Injection), Iron Sucrose Injection (Venofer), Istodax (Romidepsin for Injection), Itraconazole Injection (Sporanox Injection), Jevtana (Cabazitaxel Injection), Jonexa, Kalbitor (Ecallantide Injection), KCL in D5NS (Potassium Chloride in 5% Dextrose and Sodium Chloride Injection), KCL in D5W, KCL in NS, Kenalog 10 Injection (Triamcinolone Acetonide Injectable Suspension), Kepivance (Palifermin), Keppra Injection (Levetiracetam), Keratinocyte, KFG, Kinase Inhibitor, Kineret (Anakinra), Kinlytic (Urokinase Injection), Kinrix, Klonopin (clonazepam), Kytril Injection (Granisetron Hydrochloride), lacosamide Tablet and Injection (Vimpat), Lactated Ringer's, Lanoxin Injection (Digoxin Injection), Lansoprazole for Injection (Prevacid I.V.), Lantus, Leucovorin Calcium (Leucovorin Calcium Injection), Lente (L), Leptin, Levemir, Leukine Sargramostim, Leuprolide Acetate, Levothyroxine, Levetiracetam (Keppra Injection), Lovenox, Levocarnitine Injection (Carnitor Injection), Lexiscan (Regadenoson Injection), Lioresal Intrathecal (Baclofen Injection), Liraglutide [rDNA] Injection (Victoza), Lovenox (Enoxaparin Sodium Injection), Lucentis (Ranibizumab Injection), Lumizyme, Lupron (Leuprolide Acetate Injection), Lusedra (Fospropofol Disodium Injection), Maci, Magnesium Sulfate (Magnesium Sulfate Injection), Mannitol Injection (Mannitol IV), Marcaine (Bupivacaine Hydrochloride and Epinephrine Injection), Maxipime (Cefepime Hydrochloride for Injection), MDP Multidose Kit of Technetium Injection (Technetium Tc99m Medronate Injection), Mecasermin [rDNA origin] Injection (Increlex), Mecasermin Rinfabate [rDNA origin] Injection (Iplex), Melphalan Hcl Injection (Alkeran Injection), Methotrexate, Menactra, Menopur (Menotropins Injection), Menotropins for Injection (Repronex), Methohexital Sodium for Injection (Brevital Sodium), Methyldopate Hydrochloride Injection, Solution (Methyldopate Hcl), Methylene Blue (Methylene Blue Injection), Methylprednisolone Acetate Injectable Suspension (Depo Medrol), MetMab, Metoclopramide Injection (Reglan Injection), Metrodin (Urofollitropin for Injection), Metronidazole Injection (Flagyl Injection), Miacalcin, Midazolam (Midazolam Injection), Mimpara (Cinacalet), Minocin Injection (Minocycline Inj), Minocycline Inj (Minocin Injection), Mipomersen, Mitoxantrone for Injection Concentrate (Novantrone), Morphine Injection (Duramorph), Morphine Sulfate XR Liposome Injection (DepoDur), Morrhuate Sodium (Morrhuate Sodium Injection), Motesanib, Mozobil (Plerixafor Injection), Multihance (Gadobenate Dimeglumine Injection), Multiple Electrolytes and Dextrose Injection, Multiple Electrolytes Injection, Mylotarg (Gemtuzumab Ozogamicin for Injection), Myozyme (Alglucosidase alfa), Nafcillin Injection (Nafcillin Sodium), Nafcillin Sodium (Nafcillin Injection), Naltrexone XR Inj (Vivitrol), Naprosyn (naproxen), NeoProfen (Ibuprofen Lysine Injection), Nandrol Decanoate, Neostigmine Methylsulfate (Neostigmine Methylsulfate Injection), NEO-GAA, NeoTect (Technetium Tc 99m Depreotide Injection), Nephramine (Essential Amino Acid Injection), Neulasta (pegfilgrastim), Neupogen (Filgrastim), Novolin, Novolog, NeoRecormon, Neutrexin (Trimetrexate Glucuronate Inj), NPH (N), Nexterone (Amiodarone HCl Injection), Norditropin (Somatropin Injection), Normal Saline (Sodium Chloride Injection), Novantrone (Mitoxantrone for Injection Concentrate), Novolin 70/30 Innolet (70% NPH, Human Insulin Isophane Suspension and 30% Regular, Human Insulin Injection), NovoLog (Insulin Aspart [rDNA origin] Inj), Nplate (romiplostim), Nutropin (Somatropin (rDNA origin) for Inj), Nutropin AQ, Nutropin Depot (Somatropin (rDNA origin) for Inj), Octreotide Acetate Injection (Sandostatin LAR), Ocrelizumab, Ofatumumab Injection (Arzerra), Olanzapine Extended Release Injectable Suspension (Zyprexa Relprevv), Omnitarg, Omnitrope (Somatropin [rDNA origin] Injection), Ondansetron Hydrochloride Injection (Zofran Injection), OptiMARK (Gadoversetamide Injection), Optiray Injection (Ioversol Injection), Orencia, Osmitrol Injection in A viva (Mannitol Injection in Aviva Plastic Vessel 250), Osmitrol Injection in Viaflex (Mannitol Injection in Viaflex Plastic Vessel 250), Osteoprotegrin, Ovidrel (Choriogonadotropin Alfa Injection), Oxacillin (Oxacillin for Injection), Oxaliplatin Injection (Eloxatin), Oxytocin Injection (Pitocin), Paliperidone Palmitate Extended-Release Injectable Suspension (Invega Sustenna), Pamidronate Disodium Injection (Pam idronate Disodium Injection), Panitumumab Injection for Intravenous Use (Vectibix), Papaverine Hydrochloride Injection (Papaverine Injection), Papaverine Injection (Papaverine Hydrochloride Injection), Parathyroid Hormone, Paricalcitol Injection Fliptop Vial (Zemplar Injection), PARP Inhibitor, Pediarix, PEGlntron, Peginterferon, Pegfilgrastim, Penicillin G Benzathine and Penicillin G Procaine, Pentetate Calcium Trisodium Inj (Ca-DTPA), Pentetate Zinc Trisodium Injection (Zn-DTPA), Pepcid Injection (Famotidine Injection), Pergonal, Pertuzumab, Phentolamine Mesylate (Phentolamine Mesylate for Injection), Physostigmine Salicylate (Physostigmine Salicylate (injection)), Physostigmine Salicylate (injection) (Physostigmine Salicylate), Piperacillin and Tazobactam Injection (Zosyn), Pitocin (Oxytocin Injection), Plasma-Lyte 148 (Multiple Electrolytes Inj), Plasma-Lyte 56 and Dextrose (Multiple Electrolytes and Dextrose Injection in Viaflex, Plastic Vessel 250), PlasmaLyte, Plerixafor Injection (Mozobil), Polidocanol Injection (Asclera), Potassium Chloride, Pralatrexate Solution for Intravenous Injection (Folotyn), Pramlintide Acetate Injection (Symlin), Premarin Injection (Conjugated Estrogens for Injection), Prep kit for Technetium Tc99 Sestamibi for Injection (Cardiolite), Prevacid I.V. (Lansoprazole for Injection), Primaxin I.V. (Imipenem and Cilastatin for Injection), Prochymal, Procrit, Progesterone, ProHance (Gadoteridol Injection Solution), Prolia (Denosumab Injection), Promethazine HCl Injection (Promethazine Hydrochloride Injection), Propranolol Hydrochloride Injection (Propranolol Hydrochloride Injection), Quinidine Gluconate Injection (Quinidine Injection), Quinidine Injection (Quinidine Gluconate Injection), R-Gene 10 (Arginine Hydrochloride Injection), Ranibizumab Injection (Lucentis), Ranitidine Hydrochloride Injection (Zantac Injection), Raptiva, Reclast (Zoledronic Acid Injection), Recombivarix HB, Regadenoson Injection (Lexiscan), Reglan Injection (Metoclopramide Injection), Remicade, Renagel, Renvela (Sevelamer Carbonate), Repronex (Menotropins for Injection), Retrovir IV (Zidovudine Injection), rhApo2L/TRAIL, Ringer's and 5% Dextrose Injection (Ringers in Dextrose), Ringer's Injection (Ringers Injection), Rituxan, Rituximab, Rocephin (ceftriaxone), Rocuronium Bromide Injection (Zemuron), Roferon-A (interferon alfa-2a), Romazicon (flumazenil), Romidepsin for Injection (Istodax), Saizen (Somatropin Injection), Sandostatin LAR (Octreotide Acetate Injection), Sclerostin Ab, Sensipar (cinacalcet), Sensorcaine (Bupivacaine HCl Injections), Septocaine (Articane HCl and Epinephrine Injection), Serostim LQ (Somatropin (rDNA origin) Injection), Simponi Injection (Golimumab Injection), Sodium Acetate (Sodium Acetate Injection), Sodium Bicarbonate (Sodium Bicarbonate 5% Injection), Sodium Lactate (Sodium Lactate Injection in AVIVA), Sodium Phenylacetate and Sodium Benzoate Injection (Ammonul), Somatropin (rDNA origin) for Inj (Nutropin), Sporanox Injection (Itraconazole Injection), Stelara Injection (Ustekinumab), Stemgen, Sufenta (Sufentanil Citrate Injection), Sufentanil Citrate Injection (Sufenta), Sumavel, Sumatriptan Injection (Alsuma), Symlin, Symlin Pen, Systemic Hedgehog Antagonist, Synvisc-One (Hylan G-F 20 Single Intra-articular Injection), Tarceva, Taxotere (Docetaxel for Injection), Technetium Tc 99m, Telavancin for Injection (Vibativ), Temsirolimus Injection (Torisel), Tenormin I.V. Injection (Atenolol Inj), Teriparatide (rDNA origin) Injection (Forteo), Testosterone Cypionate, Testosterone Enanthate, Testosterone Propionate, Tev-Tropin (Somatropin, rDNA Origin, for Injection), tgAAC94, Thallous Chloride, Theophylline, Thiotepa (Thiotepa Injection), Thymoglobulin (Anti-Thymocyte Globulin (Rabbit), Thyrogen (Thyrotropin Alfa for Injection), Ticarcillin Disodium and Clavulanate Potassium Galaxy (Timentin Injection), Tigan Injection (Trimethobenzamide Hydrochloride Injectable), Timentin Injection (Ticarcillin Disodium and Clavulanate Potassium Galaxy), TNKase, Tobramycin Injection (Tobramycin Injection), Tocilizumab Injection (Actemra), Torisel (Temsirolimus Injection), Totect (Dexrazoxane for Injection, Intravenous Infusion Only), Trastuzumab-DM1, Travasol (Amino Acids (Injection)), Treanda (Bendamustine Hydrochloride Injection), Trelstar (Triptorelin Pamoate for Injectable Suspension), Triamcinolone Acetonide, Triamcinolone Diacetate, Triamcinolone Hexacetonide Injectable Suspension (Aristospan Injection 20 mg), Triesence (Triamcinolone Acetonide Injectable Suspension), Trimethobenzamide Hydrochloride Injectable (Tigan Injection), Trimetrexate Glucuronate Inj (Neutrexin), Triptorelin Pamoate for Injectable Suspension (Trelstar), Twinject, Trivaris (Triamcinolone Acetonide Injectable Suspension), Trisenox (Arsenic Trioxide Injection), Twinrix, Typhoid Vi, Ultravist (Iopromide Injection), Urofollitropin for Injection (Metrodin), Urokinase Injection (Kinlytic), Ustekinumab (Stelara Injection), Ultralente (U), Valium (diazepam), Valproate Sodium Injection (Depacon), Valtropin (Somatropin Injection), Vancomycin Hydrochloride (Vancomycin Hydrochloride Injection), Vancomycin Hydrochloride Injection (Vancomycin Hydrochloride), Vaprisol (Conivaptan Hcl Injection), VAQTA, Vasovist (Gadofosveset Trisodium Injection for Intravenous Use), Vectibix (Panitumumab Injection for Intravenous Use), Venofer (Iron Sucrose Injection), Verteporfin Inj (Visudyne), Vibativ (Telavancin for Injection), Victoza (Liraglutide [rDNA] Injection), Vimpat (lacosamide Tablet and Injection), Vinblastine Sulfate (Vinblastine Sulfate Injection), Vincasar PFS (Vincristine Sulfate Injection), Victoza, Vincristine Sulfate (Vincristine Sulfate Injection), Visudyne (Verteporfin Inj), Vitamin B-12, Vivitrol (Naltrexone XR Inj), Voluven (Hydroxyethyl Starch in Sodium Chloride Injection), Xeloda, Xenical (orlistat), Xeomin (Incobotulinumtoxin A for Injection), Xolair, Zantac Injection (Ranitidine Hydrochloride Injection), Zemplar Injection (Paricalcitol Injection Fliptop Vial), Zemuron (Rocuronium Bromide Injection), Zenapax (daclizumab), Zevalin, Zidovudine Injection (Retrovir IV), Zithromax Injection (Azithromycin), Zn-DTPA (Pentetate Zinc Trisodium Injection), Zofran Injection (Ondansetron Hydrochloride Injection), Zingo, Zoledronic Acid for Inj (Zometa), Zoledronic Acid Injection (Reclast), Zometa (Zoledronic Acid for Inj), Zosyn (Piperacillin and Tazobactam Injection), Zyprexa Relprevv (Olanzapine Extended Release Injectable Suspension) and combinations thereof.

Preferably, the first fluid flow path is free of a microbial filter. By lacking a microbial filter the first fluid flow path may be used to withdraw CSF that may have cells or other large molecules without such cells or large molecules being filtered. The first fluid flow path may also be used to introduce therapeutic fluids that comprise cells or other large molecules.

The second fluid flow path may comprise a microbial filter. The microbial filter may be positioned at any suitable location of the second fluid flow path.

Referring now to **FIG. 15****,** an implantable cranial device **100** of the invention includes a first fluid flow path **192** extending from a first opening **116** in the top surface **112** of the device **100** to a first opening **126** at the bottom of the device **100.** The implantable cranial device **100** includes a second fluid flow path **194** extending from a second opening **118** in an upper flange portion of the device **100** to a second opening **128** at the bottom of the device **100.** The second upper flange portion opening **118** in the depicted embodiment is defined by an external catheter connector **140.** The first **126** and second **128** openings of the bottom of the device are formed by a brain catheter connector **130.** The filter **499** is depicted as being in the second pathway **194** in proximity to the external catheter connector **140.** However, the filter **499** may be positioned at any suitable location in the second pathway **194.**

### System

The implantable cranial medical devices described herein and associated devices, such as brain catheters, external catheters, and the like may be used with any suitable external device or external devices. The external devices may be infusion devices. The infusion device may be implantable or non-implantable. The non-implantable infusion device may be an ambulatory device or a stationary device. The infusion device may be manually powered, electromechanically powered, chemically powered, or otherwise powered. In some examples, the infusion device may comprise a piston pump, a peristaltic pump, an osmotic pump, a plunger, or the like. A distal portion of the CSF shunt or drainage catheter may be placed in any suitable location of the patient, such as the peritoneal cavity.

In some embodiments, more than one external device is connected to the implantable cranial medical device in serial. For example, the devices may be "daisy-chained". In some examples, a passive infusion device is fluidly positioned between the implantable cranial medical device and an active infusion device, such a powered infusion device.

The passive device may be a modified Ommaya or Rickman reservoir. The reservoirs may be modified such that they do not need to be coupled to a catheter implanted in the brain. Rather the reservoirs may be operably coupled to the implantable cranial medical device, such as through the second fluid path, to deliver therapeutic fluid to the brain via the second lumen of the brain catheter. The reservoirs may be implanted under the scalp. The reservoirs may contain ports for introducing needles percutaneously to deliver therapeutic fluids. The reservoirs may comprise appropriate valves to ensure one-way direction of flow from the reservoir to the implantable cranial medical device.

The use of such reservoirs may be beneficial if multiple manual infusions of therapeutic agent are anticipated. Rather than repeatedly percutaneously accessing the port of the implantable cranial medical device to both aspirate CSF and infuse therapeutic fluid, the reservoir may be used introduce the therapeutic fluid, thereby reducing the number of times the skin in punctured over the port of the implantable cranial medical device.

The passive infusion device or port may be fully implantable, partially implantable or positioned external to a subject.

The port may be placed at a location to allow convenient access for introduction of therapeutic fluid into the CSF-containing space of the subject. For example, the second port may be located in proximity to the subject's ear ("auricular") or in proximity to the subject's clavicle ("clavicular"). The port may be configured to be implanted subcutaneously on the skull in proximity to an ear or in implanted subcutaneously in proximity to a clavicle.

The port may be used to introduce a fluid to a CSF-containing space via the implantable cranial medical device. The fluid may be a therapeutic fluid. The port may be used to deliver the therapeutic fluid until the therapy is determined to be effective. For example, the port may be used to determine an appropriate therapeutic agent for use in the therapeutic fluid, an appropriate dose range, or the like. Once the therapy is determined effective, an infusion device may be operatively coupled to the implantable cranial medical device to deliver the therapeutic fluid to the brain, e.g., to a CSF-containing space. The port may be replaced with the infusion device. Alternatively, the port and the infusion device may both be operatively coupled with the implantable cranial medical device.

Some therapies involve periodic, rather than continuous, infusion of therapeutic fluid. For such therapies, the port may be used to chronically deliver periodic infusions of the therapeutic fluid to a CSF-containing space via the implantable cranial medical device.

Some therapies involve infusion of more than one therapeutic fluid. For such therapies, an infusion device operatively coupled to the implantable cranial medical device may be used to deliver a first therapeutic fluid to a CSF-containing space via the implantable cranial medical device, and the port may be used deliver a second a second therapeutic fluid to the CSF-containing space via the implantable cranial medical device. The port may be used to deliver auxiliary therapeutic fluids, to treat symptoms that may arise during infusion of the first therapeutic fluid, or the like.

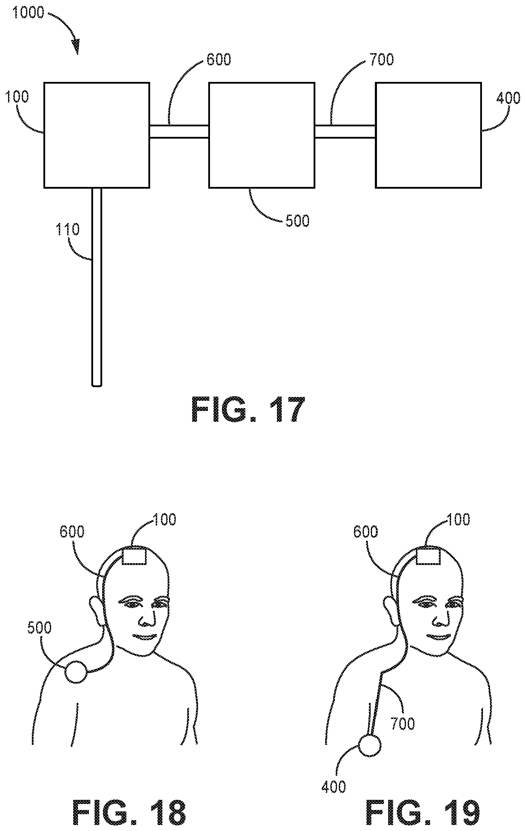

Referring now to **FIGS. 16** and **17****,** schematic drawings of systems **1000** are shown. The systems include an implantable cranial medical device **100** and associated brain catheter **200.** The system **1000** also includes an external catheter **600** operably coupled to a first **400** or second **500** external device. In **FIG. 16****,** the implantable cranial medical device **100** is connected to the first device **400** via the external catheter **600.** In **FIG. 17****,** the implantable cranial medical device **100** is connected to the second device **500** via the external catheter **600,** and the second device **500** is connected to the first device **400** via an additional catheter **700.** The first device **400** may be an implantable infusion device, such as a powered implantable infusion device. The second device **500** may be a reservoir having a port accessible through the scalp of a subject, such as a modified Ommaya or Rickman reservoir.

Reference is now made to **FIGS. 18-21****,** which illustrate some components of embodiments of systems described herein. **FIG. 18** shows an implantable cranial medical device **100** implanted in a subject's head and a port **500** in proximity to the subject's clavicle. The port **500** may be implanted or may be external to the subject. The implantable cranial medical device **100** and port **500** may be implanted in, or positioned in proximity to, any other suitable location of the subject. A catheter **600** operatively couples the implantable cranial medical device **100** to the port **500.**

In **FIG. 19****,** the port **500** is disconnected from the catheter **600,** and an infusion device catheter **700** that is operatively coupled to an infusion device **400** is coupled to catheter **600.** The infusion device catheter **700** may be coupled to catheter **600** in any suitable manner. For example, a splice connector (not shown) having components similar to those described above regarding catheter connectors may be employed to couple the infusion device catheter **700** to catheter **600.** Preferably, the infusion device **400** is implanted in the subject.

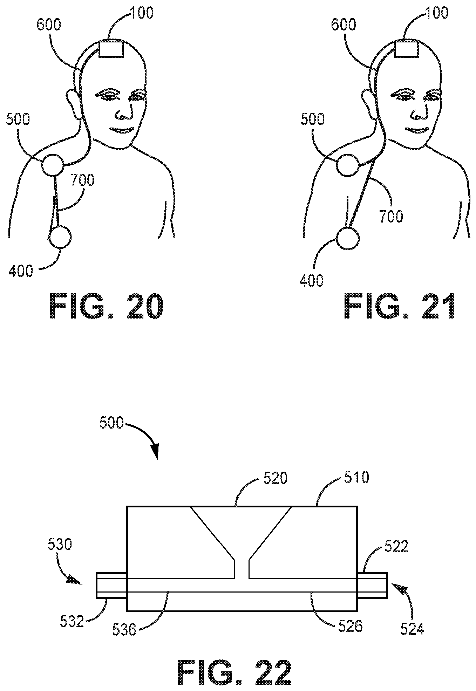

**FIG. 20** illustrates an embodiment where an infusion device **400** is operatively coupled to the port **500** via an infusion device catheter **700.** The infusion device catheter **700** may be coupled to an additional inlet (not shown), such as via a catheter connector (not shown), of the port **500.** Preferably, the port **500** and the infusion device **400** are implanted.

**FIG. 21** illustrates an embodiment in which an infusion device **400** is operatively coupled to catheter **600** via an infusion device catheter **700** while the port **500** is also coupled to catheter **600.** Catheter **600** may be bifurcated at its proximal end portion to permit both the port **500** and the infusion device **400** to be simultaneously operatively coupled to the catheter **600.** Alternatively, a Y-adapter or T-adapter (not shown) having components such as those described above regarding catheter connectors may be employed to operatively couple both the port **500** and the infusion device **400,** via the infusion device catheter **700,** to catheter **600.**

**FIG. 22** illustrates an embodiment of a port **500** configured to couple to an infusion device **400** and the implantable cranial medical device. The port **500** includes a housing **510** that defines at least a portion of the outer surface of the second port. The housing **510** may define an opening that serves as the third inlet **520.** A self-sealing septum (not shown) may be disposed across the opening defining the third inlet **520.** The self-sealing septum may be as described above regarding a self-sealing septum of the implantable cranial medical device. The third inlet **520** may comprise a ferrule (not shown) and compression wedge (not shown) as described above regarding the catheter connector of the implantable cranial medical device.

The port **500** may comprise a catheter connector **522** that defines an outlet **524.** The port **500** comprises a fluid pathway **526** that extends from an inlet **520** to the outlet **524.** The catheter connector **522** may positioned at any suitable location of the second port **500.** In **FIG. 22****,** the catheter connector **522** is positioned at a side surface of the port **500.** The catheter connector **522** is configured to operatively couple to a catheter to place a lumen of the catheter in fluid communication with the fluid pathway **526.** The catheter may also be coupled to the external catheter connector of the implantable cranial medical device. The catheter connector **522** of the port **500** may comprise components of a catheter connector as described above regarding the implantable cranial medical device.

The port **500** may comprise an infusion device catheter connector **532.** The infusion device catheter connector **532** defines an inlet **530.** A fluid pathway **536** extends from the inlet **530** to the outlet **524.** The infusion device catheter connector **532** is configured to operatively couple to an infusion device catheter to place a lumen of the infusion device catheter in fluid communication with the fluid pathway **536.** The infusion device catheter connector **522** of the port **500** may comprise components of a catheter connector as described above regarding the implantable cranial medical device.

### Device and system for delivering to fluid to or withdrawing from intrathecal space

The devices, systems, kits and methods described above are primarily described in the context of their use for delivering therapeutic fluids directly to the brain or withdrawing CSF from a region of the brain. It will be understood that the implantable cranial medical devices, catheters, systems, kits, and methods described herein may be used to deliver therapeutic agents to, or withdraw CSF from, the intrathecal space. The disclosed methods do not form part of the invention.

The devices, systems and devices, systems, kits and methods may be modified, as appropriate to deliver fluid to the intrathecal space. For example, the implantable cranial medical device may be modified for implanting in fascia rather than under the scalp of a subject. The device may be implanted in any suitable location of the patient, such as in the torso of the subject, near the back of the neck of the subject, or the like. The disclosed methods do not form part of the invention.

The shape of the device may be modified to accommodate the site of implantation. While the device may not maintain the shape, size and dimensions of the implantable cranial medical device described above, the functionality of the device may remain largely unchanged. That is, the device may comprise a first and second fluid flow paths and connector for connecting one or more catheters (referred hereinafter as a "spinal catheter"), preferably a multi-lumen spinal catheter, that may be placed in communication with the intrathecal space. The device may comprise a port into which needles may be inserted to deliver therapeutic fluid to, or withdraw CSF from, the intrathecal space via the first fluid flow path and first lumen of a spinal catheter. The device may comprise external catheter connector for connecting to an external catheter to place a lumen of the external catheter in communication with the second fluid flow path. The external catheter may be used to connect to an external device or for draining CSF.

The length of a spinal catheter may be greater than the length of a brain catheter described above. The spinal catheter may be positioned in any suitable location of the intrathecal space. Preferably, the distal tip of the spinal catheter is positioned at cervical vertebral level or higher. In some embodiments, the distal tip of the spinal catheter is advanced through the intrathecal space into the cisterna magna.

The spinal catheter may have a length sufficient to be inserted into the intrathecal space and to be advanced to a higher vertebral level or the cisterna magna. The spinal catheter may be configured to enter the intrathecal space at any suitable vertebral level. For example, the spinal catheter may be configured to be introduced into the intrathecal space at a lumbar vertebral level.

The spinal catheter may have an outer diameter greater than the outer diameter of a brain catheter described above.

The spinal catheter may have multiple openings in fluid communication with an aspiration fluid flow path of the device accessible via the port. The multiple openings may be positioned any suitable distance apart, such as about three inches to about seven inches, to allow for sampling of CSF at different locations along the spine, as different biomarkers or concentrations may be present at different CSF locations. The spinal catheter may have any suitable number of openings, such as three, four, five, or more.

In some embodiments, the port has multiple aspiration fluid flow paths with each aspiration path separately coupled with a lumen of a catheter or catheters. If the catheter is a multi-lumen catheter the distal end portion of the lumens may be separated by about three inches to about seven inches along the length of the catheter so that CSF may be sampled along the length of the spinal canal. The multi-lumen spinal catheter may have any suitable number of aspiration lumens, such as three, four, five or more.

In some embodiments, the device may have multiple spinal catheter connectors with each spinal catheter connector configured to connect to a separate spinal catheter. In some embodiments, the first fluid path (the path in communication with the port) is in communication with more than one catheter port. Spinal catheters having different lengths may be coupled to the spinal catheter connectors, so that the catheters may be positioned within the intrathecal space at different vertebral levels for aspirating CSF along the length of the spinal canal.

The materials described above for the implantable cranial medical device, brain catheter, and other system and kit components may be the same for the device and spinal catheter for delivering therapeutic fluid to, or withdrawing fluid from, the intrathecal space.

The methods discussed above regarding the use of the implantable cranial medical device, systems and kits may be employed using a device, system, or kit adapted for delivering therapeutic fluid to, or withdrawing fluid from, the intrathecal space. These methods do not form part of the invention.

All scientific and technical terms used herein have meanings commonly used in the art unless otherwise specified. The definitions provided herein are to facilitate understanding of certain terms used frequently herein and are not meant to limit the scope of the present disclosure.

As used herein, singular forms "a," "an" and "the" include plural referents unless the context clearly dictates otherwise. As used in this specification and the appended claims, the term "or" is generally employed in its sense including "and/or" unless the content clearly dictates otherwise. The term "and/or" means one or all of the listed elements or a combination of any two or more of the listed elements.

The words "preferred" and "preferably" refer to embodiments of the disclosure that may afford certain benefits, under certain circumstances. However, other embodiments may also be preferred, under the same or other circumstances. Furthermore, the recitation of one or more preferred embodiments does not imply that other embodiments are not useful and is not intended to exclude other embodiments from the scope of the inventive technology.

Any direction referred to herein, such as "top," "bottom," "side," "upper," "lower," and other directions or orientations are described herein for clarity and brevity but are not intended to be limiting of an actual device or system. Devices and systems described herein may be used in a number of directions and orientations.

The headings used herein are for organizational purposes only and are not meant to be used to limit the scope of the description. As used throughout this application, the word "may" is used in a permissive sense (i.e., meaning having the potential to), rather than the mandatory sense (i.e., meaning must).

The words "include," "including," and "includes" indicate open-ended relationships and therefore mean including, but not limited to. Similarly, the words "have," "having," and "has" also indicated open-ended relationships, and thus mean having, but not limited to. Similarly, the terms "comprise" and "comprising" indicate open-ended relationships, and thus mean comprising, but not limited to. The terms "consisting essentially of" and "consisting of" are subsumed within the term "comprising." For example, a catheter comprising tubing may be a catheter consisting of tubing. The term "consisting essentially of" means a recited list of one or more items belonging to an article, kit, system, or method and other non-listed items that do not materially affect the properties of the article, kit, system, or method.

The terms "first," "second," "third," and so forth as used herein are used as labels for nouns that they precede, and do not imply any type of ordering (e.g., spatial, temporal, logical, etc.) unless such an ordering is otherwise explicitly indicated. For example, a "second" feature does not require that a "first" feature be implemented prior to the "second" feature, unless otherwise specified.

Various components may be described as "configured to" perform a task or tasks. In such contexts, "configured to" is a broad recitation generally meaning "having structure that" performs the task or tasks during operation. As such, the component can be configured to perform the task even when the component is not currently performing that task (e.g., a catheter connector may be configured to place a lumen of a catheter in fluid communication with a fluid path, even when the catheter is not connected to the catheter connector).

Provided below are non-limiting examples illustrating certain aspects or features of the present disclosure, which do not form part of the invention.

### EXAMPLES

### Example 1: Prophetic Implantation Procedure (not part of the invention)

An example of an implant procedure that may be used to implant an implantable cranial medical device and associated devices is described in this example. The implantable cranial medical device may be packaged with a dual lumen brain catheter and accessories, such as screws, tools for use with the screws, an external catheter compression fitting, and the like. Other generally available accessories to add in the navigation and implantation are specified here but not included in the Kit. The implantable cranial medical device may be compatible with and intended to be used with the AxiEM^{™} electromagnetic technology for StealthStation^{®} Navigation System (Medtronic Inc.) and the Intraventricular Disposable Introducer Set^{™} (B. Braun Aesculap division). The trajectory site of origin on the skull and length catheter may be finalized during the surgical planning with the navigation system.

The trajectory may be made with the aid of a 10 French Intraventricular Disposable Introducer (also known as Peel Away Sheath) which facilitates the process of aiming and introducing the AxiEM navigation stylet, and (secondly) the brain catheter, into the lateral ventricle. The depth scaling on the Disposable Introducer increases the security and control while introducing the AxiEM stylet. The Sheath of the introducer protects the cerebral tissue while inserting and withdrawing the AxiEM stylet during navigation. The round and blunt obturator tip on the Disposable Introducer ensures less traumatic insertion into the brain. The Disposable Introducer is also easy to peel with side handles once the catheter is in place.

After the trajectory is set with the Disposable introducer and AxiEM stylet, the Cranial Port, connected to the cranial catheter, may be lowered into the inner lumen of the temporary sheath. The sheath may then be removed. The upper flange portion of the implantable cranial medical device may be fastened to the skull with cranial screws. The external catheter may be connected to the port after it is tunneled from a separate infusion device, such as an implantable pump. A compression fitting may be slid over the external catheter towards the implantable cranial medical device to securely retain the external catheter relative to the implantable cranial medical device.

The following list provides some additional details:
1. Subjects will undergo a preoperative high-resolution MRI or CT scan of the brain for surgical planning.
2. Subject is positioned supine on the operating table and the head is turned as needed for Stealth AxiEM utilization.
3. Stealth AxiEM System is registered to the patient and multiple head touch points are utilized as part of the setup and registration of the stealth. Using the Stealth planning software, the location for the tip of the brain catheter after placement is chosen to be 1 cm above and 1 cm proximal to the ipsilateral foramen of Monro. The entry point is chosen to be Kochers Point via anatomic landmarks and by referencing imaging. The resultant trajectory is then reviewed and confirmed to avoid sulcal boundaries and cortical vessels and planned to traverse a gyrus. Small modifications should be made to the final entry point to facilitate this goal.
4. A curvilinear incision is made over the entry point for the port to be placed under the scalp allowing for room to tunnel from the abdomen the proximal pump tubing using standard technique.
5. A burr hole is placed over the identified entry point using a standard 14mm perforator and the dura is cauterized. If an implantable infusion device and associated external catheter are used, the manufacturer's instructions for implanting the infusion device and tunneling the external catheter should be followed.
6. AxiEM Stylet (9733605 AxiEM^{™} Non-invasive shunt kit, Medtronic Inc.) is placed in the obturator of a 10 French Intraventricular Disposable Introducer Sheath (PaediScope^{™} PF010A, B. Braun)- Place Obturator/AxiEM within the sheath. A suture is placed around the AxiEM to enable maximum penetration during advancement to target but does not proceed beyond planned target location in the ventricle. During placement of AxiEM in sheath in to target location, endeavor to minimize CSF loss as possible.
7. Confirm trajectory guidelines and target guidelines are met (see above #4)
8. The dura is opened in a cruciate fashion and leaflets are cauterized. Pia arachnoid is cauterized and opened.
9. Connect disposable tubing to external catheter connector on implantable cranial medical device. Flush second fluid path of implantable cranial medical device with 0.250 milliliters per second slowly over 2 minutes. Remove disposable tubing and connect external catheter to external catheter connector.
10. The sheath and AxiEM stylet are advanced towards the target using navigation trajectory and guidance views and the sheath and Axiem left in place just at the target point.
11. The two leafs of the sheath are peeled back and secured to the drape and care is taken to avoid movement of the sheath or the AxiEM tip within the brain.
12. The connected implantable cranial medical device and dual lumen brain catheter are prepped for insertion.
13. At the same time, the AxiEM and obturator is removed, and the sheath left in place just before the target point. The dual lumen brain catheter is placed within the sheath to the depth of the target location.
14. The upper flange portion of the implantable cranial medical device is held against the skull.
15. Loss of CSF is estimated and recorded after CSF is gently aspirated if needed as per the usual technique for sampling CSF from a shunt or an ommaya port. Send specimen as needed for routine CSF labs (anticipate > 2 milliliters of CSF total).
16. The peel away sheath is removed while holding the cranial port against bone.
17. Install self-tapping cranial screws around the implantable cranial medical device and burr hole to secure the implantable cranial medical device to the skull.
18. Confirm proper placement, that the tip of double lumen brain catheter is in the ventricle, by one or both of CSF return and imaging including CT scan, O-arm or contrast with fluoro.
19. Verify integrity of ligatures at all junctions to prevent obstruction of the external catheter lumen and tears or abrasions of the tubing of external catheter.
20. Suture skin.

### Example 2: Prophetic Aspiration Procedure (not part of the invention)

An example of a procedure that may be used to aspirate CSF is provided in this example. The procedure includes:
1. After providing the purpose, risks and benefits, and steps of the procedure obtain informed consent from the patient or appropriate legal designee.
2. Wash hands. Don sterile gloves.
3. Scrub site with chlorhexadine or per site standard operating procedure (SOP).
4. Insert a 1,7 mm diameter (25-gauge) butterfly needle directly into the port of the implantable cranial medical device at a perpendicular angle to skin. Angle needle at 30 - 45 degrees from the skin and avoid being up against the skin.
5. Aspirate fluid into syringe slowly (not more than 1 ml per minute).
6. Limit the total volume aspirated at each tapping to no more than 7 ml. The initial puncture should not exceed 7 ml in volume and not more than 7 ml/day.
7. Send sample for culture, cell count, glucose, protein, and other biomarkers as requested by physician.
21. Remove needle and hold firm pressure for 2 minutes or until CSF leakage from skin stops. Clean chlorhexadine from the skin after the procedure.

Following the procedure, the subject should be assessed for possible side-effects. For example, the subject should be observed for signs of the following complications: local skin breakdown; intravascular fluid depletion; hypoproteinemia; hyponatremia, wound or device infections; and CSF leak from puncture site.

The following should be documented: pretreatment evaluation, informed consent, timeout, procedure, including type and size of needle, patient response, characteristics of CSF, amount of CSF withdrawn, what tests ordered on specimens, subject follow-up instructions including anti-emetics if necessary, as well as any complications.

All abnormal or unexpected laboratory findings should be reviewed by a physician.

### Example 3: ICVRx implantable cranial device from Cerebral Therapeutics, Inc. (not part of the invention)

The implantable cranial medical devices described herein may be a part of an infusion system that enables intracerebroventricular (ICV) delivery of therapeutic agents, including investigational medicines, into the cerebrospinal fluid (CSF) for treatment of diseases of the central nervous system (CNS). The devices may also be used for withdrawal of CSF for suitable purposes, such as biomarker, drug or drug metabolite concentration, routine laboratory and safety analysis. The complete infusion system in clinical use may include an implantable pump and pump catheter, joined together with the implantable cranial medical device and dual lumen cranial catheter.

One such implantable cranial implantable medical device and dual lumen catheter is the ICVRx Cranial Port and Dual Lumen Cranial Catheter from Cerebral Therapeutics, Inc. The ICVRx Cranial Port and Dual Lumen Cranial Catheter is currently under investigational use and includes a machined titanium access port and dual lumen intracranial catheter made from radiopaque tubing with a an embedded radiopaque (tantalum) tip. The dual function of the ICVRx Cranial Port and Dual Lumen Cranial Catheter supports continuous infusion of therapeutic agents into, and periodic aspiration of CSF out of, the lateral ventricle of the brain. Aspirated CSF may be used to monitor for early signs of infection (e.g., meningitis), drug concentration, markers of disease progression, and the like. The ICVRx Cranial Port and Dual Lumen Cranial Catheter is designed to have the advantages of both an Ommaya reservoir and a catheter fixation device for CNS infusion in a single small, low profile implant that can be placed efficiently and accurately by a neurosurgical team. The cranial dual lumen catheter has radiopaque characteristics to aid in localization by imaging after surgical placement.

The design and construction of the ICVRx Cranial Port and Dual Lumen Cranial Catheter is intended to improve on existing methods and devices including Ommaya and Rickham reservoirs. The ICVRx Cranial Port and Dual Lumen Cranial Catheter is designed to withstand long term implantation and use. As such, it should be compatible with safety and regulatory requirements for long term implant including biocompatibility, functional testing, periodic CSF sampling and robust against active patient use.

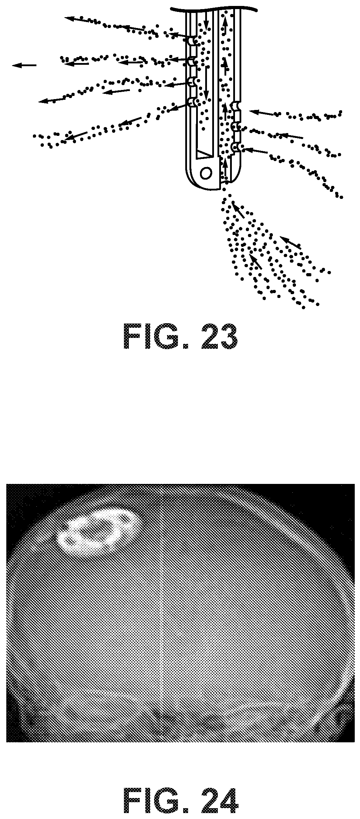

Design and construction of the ICVRx device has a focus on separating infusion of therapeutic agent from aspiration of CSF. **FIG. 23** shows separated lumens within the catheter for infusing (purple) and aspiration (green). Separation of the infusion and sampling lumens ensures no, or very little, mixing occurs and that the sample CSF represents the proportions of drug distribution actually seen in the ventricle. The ICRVx Cranial Port and Dual Lumen Cranial Catheter is made from material suitable for long term implantation such as titanium and silicone (septum), as well as a polycarbonate polyurethane for the catheter.

Non-clinical bench testing for the catheter demonstrates its ability for long term use. Testing for flow path construction included preconditioning with over 1000 cycles of catheter attachment fatigue followed by high pressure burst testing with requirements greater than 80psi for burst. In addition, after conditioning, all catheter attachment exceeded 5N detachment requirements.

For sampling the cerebrospinal fluid through the catheter, the port is built with a robust silicone reservoir. This reservoir was tested for 500 punctures with a non-coring sampling needle and still maintained a leak-free seal demonstrated by pressure testing to 80 psi after completion of punctures. This allows frequent CSF sampling while maintaining reservoir integrity and potentially reducing the infection risk.

Attachment of the ICVRx port to the skull is performed using cranial bone screws comment to orthopedic skull plating. These screws were tested to demonstrate equivalent or better performance including bone retention, torque to shear and tensile strength.

Initial implants have been performed as part of Phase 2B study. **FIG. 24** is capture as part of that study and demonstrates the radiopacity of the device under x-ray imaging. Note the port at the skull as well as the catheter path into the ventricle.

Cerebral Therapeutics' proprietary ICVRx Cranial Port and Dual Lumen Cranial Catheter is currently in use in clinical study for infusion of sodium valproate in cerebral ventricles for treatment of refractory epilepsy. The device is designed and tested to support chronic implant and long-term use for infusing therapeutics directly into the ventricle.

## Claims

1. An implantable cranial medical device (100) comprising:
a first fluid path (192);
a second fluid path (194);
an upper flange portion (110);
a lower portion (120); and
a housing (101) defining an exterior surface of the implantable cranial medical device including an exterior surface of the upper flange portion and an exterior surface of the lower portion,
wherein the upper flange portion is configured to rest on a skull of subject about a burr hole,
wherein the lower portion is configured to be placed within the burr hole,
wherein the first fluid path extends within the housing from a first opening (116) in the upper flange portion to a first opening (126) in the lower portion,
wherein the second fluid path extends within the housing from a second opening (118) in the upper flange portion to a second opening (128) in the lower portion, and
wherein the first fluid path and the second fluid path are separate and isolated.

2. The implantable cranial medical device (100) of claim 1, wherein the upper flange portion (110) has width in a range from 15 millimeters to 30 millimeters and the lower portion (120) has a width in a range from 10 millimeters to 20 millimeters and wherein the upper flange portion has a greater width than the lower portion.

3. The implantable cranial medical device (100) of claim 1 or 2, wherein the lower portion (120) has a height in a range from 3 millimeters to 7 millimeters.

4. The implantable cranial medical device (100) of any of claims 1 to 3, wherein the upper flange portion (110) has a height in a range from 3 millimeters to 8 millimeters.

5. The implantable cranial medical device (100) of any claims 1 to 4, comprising a port (150) defining an opening (116) in communication with the first fluid path (192).

6. The implantable cranial medical device (100) of claim 5, wherein the first opening (116) of the upper flange portion (110) defines the opening of the port (150),
wherein, optionally, the upper flange portion (110) comprises a top surface (112) and wherein first opening (116) of the upper flange portion is defined by the top surface.

7. The implantable cranial medical device (100) of claim 5 or 6, comprising a septum (170), wherein the septum seals the opening of the port (150).

8. The implantable cranial medical device (100) of any one of claims 1 to 7, comprising an external catheter connector (140) in communication with the second fluid path (194),
wherein, optionally, the external catheter connector (140) comprises a fitting (142) configured to be inserted into a lumen of the external catheter (600).

9. The implantable cranial medical device (100) of claim 8, wherein the external catheter connector (140) has a longitudinal axis that extends substantially parallel to a bottom (114) of the upper flange portion (110).

10. The implantable cranial medical device (100) of any one of claims 1 to 9, wherein the upper flange portion (110) of the housing (101) comprises a generally flat annular bottom (114) extending laterally relative to the lower portion (120).

11. The implantable cranial medical device (100) of any one of claims 1 to 10, comprising a brain catheter connector (130) extending from the lower portion (120), the brain catheter connector comprising a first lumen (132) in fluid communication with the first fluid path (192) and a second lumen (134) in fluid communication with the second fluid path (194).

12. A system comprising:
the implantable cranial medical device (100) of claim 11; and
a brain catheter (200) operably couplable to the brain catheter connector (130),
wherein the brain catheter comprises first (212) and second (214) lumens,
wherein the first lumen of the brain catheter is placed in fluid communication with the first lumen (132) of the brain catheter connector when the brain catheter is coupled to the brain catheter connector, and
wherein the second lumen of the brain catheter is placed in fluid communication with the second lumen (134) of the brain catheter connector when the brain catheter is coupled to the brain catheter connector.

13. A system comprising:
The implantable cranial medical device (100) of any one of claims 1 to 11 or the system of claim 12; and
an implantable infusion device (400) operably coupled to the second fluid path (194).

14. The implantable cranial medical device of claim 11, wherein the brain catheter connector (130) includes a slot (135) configured to receive a portion (215) of a dual lumen brain catheter (200) that separates a first lumen (212) of the dual lumen brain catheter from a second lumen (214) of the dual lumen brain catheter.

15. The system of claim 12 or claim 13, wherein the brain catheter connector (130) includes a slot (135) configured to receive a portion (215) of the brain catheter (200) that separates the first lumen (212) of the brain catheter from the second lumen (214) of the brain catheter.

## Patentansprüche

1. Implantierbare kraniale medizinische Vorrichtung (100), umfassend:
einen ersten Fluidweg (192);
einen zweiten Fluidweg (194);
einen oberen Flanschabschnitt (110);
einen unteren Abschnitt (120); und
ein Gehäuse (101), das eine Außenfläche der implantierbaren kranialen medizinischen Vorrichtung definiert, einschließlich einer Außenfläche des oberen Flanschabschnitts und einer Außenfläche des unteren Abschnitts,
wobei der obere Flanschabschnitt konfiguriert ist, um auf einem Schädel eines Subjekts um eine Trepanation herum zu ruhen,
wobei der untere Abschnitt konfiguriert ist, um in der Trepanation untergebracht zu sein,
wobei sich der erste Fluidweg in dem Gehäuse von einer ersten Öffnung (116) in dem oberen Flanschabschnitt zu einer ersten Öffnung (126) in dem unteren Abschnitt erstreckt,
wobei sich der zweite Fluidweg in dem Gehäuse von einer zweiten Öffnung (118) in dem oberen Flanschabschnitt zu einer zweiten Öffnung (128) in dem unteren Abschnitt erstreckt, und
wobei der erste Fluidweg und der zweite Fluidweg getrennt und isoliert sind.

2. Implantierbare kraniale medizinische Vorrichtung (100) nach Anspruch 1, wobei der obere Flanschabschnitt (110) eine Breite in einem Bereich von 15 Millimeter bis 30 Millimeter aufweist und der untere Abschnitt (120) eine Breite in einem Bereich von 10 Millimeter bis 20 Millimeter aufweist, und wobei der obere Flanschabschnitt eine größere Breite als der untere Abschnitt aufweist.

3. Implantierbare kraniale medizinische Vorrichtung (100) nach Anspruch 1 oder 2, wobei der untere Abschnitt (120) eine Höhe in einem Bereich von 3 Millimeter bis 7 Millimeter aufweist.

4. Implantierbare kraniale medizinische Vorrichtung (100) nach einem der Ansprüche 1 bis 3, wobei der obere Flanschabschnitt (110) eine Höhe in einem Bereich von 3 Millimeter bis 8 Millimeter aufweist.

5. Implantierbare kraniale medizinische Vorrichtung (100) nach einem der Ansprüche 1 bis 4, umfassend einen Anschluss (150), der eine Öffnung (116) in Kommunikation mit dem ersten Fluidweg (192) definiert.

6. Implantierbare kraniale medizinische Vorrichtung (100) nach Anspruch 5, wobei die erste Öffnung (116) des oberen Flanschabschnitts (110) die Öffnung des Anschlusses (150) definiert,
wobei, optional, der obere Flanschabschnitt (110) eine Oberseite (112) umfasst, und wobei die erste Öffnung (116) des oberen Flanschabschnitts durch die Oberseite definiert ist.

7. Implantierbare kraniale medizinische Vorrichtung (100) nach Anspruch 5 oder 6, umfassend ein Septum (170), wobei das Septum die Öffnung des Anschlusses (150) abdichtet.

8. Implantierbare kraniale medizinische Vorrichtung (100) nach einem der Ansprüche 1 bis 7, umfassend ein externes Katheterverbindungsstück (140) in Kommunikation mit dem zweiten Fluidweg (194),
wobei, optional, das externe Katheterverbindungsstück (140) einen Einbau (142) umfasst, der konfiguriert ist, um in ein Lumen des externen Katheters (600) eingeführt zu sein.

9. Implantierbare kraniale medizinische Vorrichtung (100) nach Anspruch 8, wobei das externe Katheterverbindungsstück (140) eine Längsachse aufweist, die sich im Wesentlichen parallel zu einer Unterseite (114) des oberen Flanschabschnitts (110) erstreckt.

10. Implantierbare kraniale medizinische Vorrichtung (100) nach einem der Ansprüche 1 bis 9, wobei der obere Flanschabschnitt (110) des Gehäuses (101) eine im Allgemeinen flache, ringförmige Unterseite (114) umfasst, die sich relativ zum unteren Abschnitt (120) lateral erstreckt.

11. Implantierbare kraniale medizinische Vorrichtung (100) nach einem der Ansprüche 1 bis 10, umfassend ein Hirnkatheter-Verbindungsstück (130), das sich von dem unteren Abschnitt (120) erstreckt, wobei das Hirnkatheter-Verbindungsstück ein erstes Lumen (132) in Fluidkommunikation mit dem ersten Fluidweg (192) und ein zweites Lumen (134) in Fluidkommunikation mit dem zweiten Fluidweg (194) umfasst.

12. System, umfassend:
die implantierbare kraniale medizinische Vorrichtung (100) nach Anspruch 11; und
einen Hirnkatheter (200), der operabel an das Hirnkatheter-Verbindungsstück (130) gekoppelt werden kann,
wobei der Hirnkatheter erste (212) und zweite (214) Lumen umfasst,
wobei das erste Lumen des Hirnkatheters in Fluidkommunikation mit dem ersten Lumen (132) des Hirnkatheter-Verbindungsstücks gebracht wird, wenn der Hirnkatheter an das Hirnkatheter-Verbindungsstück gekoppelt ist, und
wobei das zweite Lumen des Hirnkatheters in Fluidkommunikation mit dem zweiten Lumen (134) des Hirnkatheter-Verbindungsstücks gebracht wird, wenn der Hirnkatheter an das Hirnkatheter-Verbindungsstück gekoppelt ist.

13. System, umfassend:
die implantierbare kraniale medizinische Vorrichtung (100) nach einem der Ansprüche 1 bis 11 oder das System nach Anspruch 12; und
eine implantierbare Infusionsvorrichtung (400), die operabel an den zweiten Fluidweg (194) gekoppelt ist.

14. Implantierbare kraniale medizinische Vorrichtung nach Anspruch 11, wobei das Hirnkatheter-Verbindungsstück (130) einen Schlitz (135) beinhaltet, der konfiguriert ist, um einen Abschnitt (215) eines Doppellumen-Hirnkatheters (200) aufzunehmen, der ein erstes Lumen (212) des Doppellumen-Hirnkatheters von einem zweiten Lumen (214) des Doppellumen-Hirnkatheters trennt.

15. System nach Anspruch 12 oder Anspruch 13, wobei das Hirnkatheter-Verbindungsstück (130) einen Schlitz (135) beinhaltet, der konfiguriert ist, um einen Abschnitt (215) des Hirnkatheters (200) aufzunehmen, der das erste Lumen (212) des Hirnkatheters von dem zweiten Lumen (214) des Hirnkatheters trennt.

## Revendications

1. Dispositif médical crânien implantable (100) comprenant :
un premier trajet fluidique (192) ;
un deuxième trajet fluidique (194) ;
une portion bride supérieure (110) ;
une portion inférieure (120) ; et
un boîtier (101) définissant une surface extérieure du dispositif médical crânien implantable comportant une surface extérieure de la portion bride supérieure et une surface extérieure de la portion inférieure,
dans lequel la portion bride supérieure est configurée pour reposer sur un crâne d'un sujet autour d'un trou de trépan,
dans lequel la portion inférieure est configurée pour être placée au sein du trou de trépan,
dans lequel le premier trajet fluidique s'étend au sein du boîtier d'une première ouverture (116) dans la portion bride supérieure à une première ouverture (126) dans la portion inférieure,
dans lequel le deuxième trajet fluidique s'étend au sein du boîtier d'une deuxième ouverture (118) dans la portion bride supérieure à une deuxième ouverture (128) dans la portion inférieure, et
dans lequel le premier trajet fluidique et le deuxième trajet fluidique sont séparés et isolés.

2. Dispositif médical crânien implantable (100) selon la revendication 1, dans lequel la portion bride supérieure (110) a une largeur dans une plage de 15 millimètres à 30 millimètres et la portion inférieure (120) a une largeur dans une plage de 10 millimètres à 20 millimètres et dans lequel la portion bride supérieure a une largeur plus grande que la portion inférieure.

3. Dispositif médical crânien implantable (100) selon la revendication 1 ou 2, dans lequel la portion inférieure (120) a une hauteur dans une plage de 3 millimètres à 7 millimètres.

4. Dispositif médical crânien implantable (100) selon l'une quelconque des revendications 1 à 3, dans lequel la portion bride supérieure (110) a une hauteur dans une plage de 3 millimètres à 8 millimètres.

5. Dispositif médical crânien implantable (100) selon l'une quelconque des revendications 1 à 4, comprenant un orifice (150) définissant une ouverture (116) en communication avec le premier trajet fluidique (192).

6. Dispositif médical crânien implantable (100) selon la revendication 5, dans lequel la première ouverture (116) de la portion bride supérieure (110) définit l'ouverture de l'orifice (150),
dans lequel, éventuellement, la portion bride supérieure (110) comprend une surface de sommet (112) et dans lequel la première ouverture (116) de la portion bride supérieure est définie par la surface de sommet.

7. Dispositif médical crânien implantable (100) selon la revendication 5 ou 6, comprenant un septum (170), dans lequel le septum obture l'ouverture de l'orifice (150).

8. Dispositif médical crânien implantable (100) selon l'une quelconque des revendications 1 à 7, comprenant un raccord de cathéter externe (140) en communication avec le deuxième trajet fluidique (194),
dans lequel, éventuellement, le raccord de cathéter externe (140) comprend un adaptateur (142) configuré pour être inséré dans une lumière du cathéter externe (600).

9. Dispositif médical crânien implantable (100) selon la revendication 8, dans lequel le raccord de cathéter externe (140) a un axe longitudinal qui s'étend sensiblement parallèlement à un fond (114) de la portion bride supérieure (110).

10. Dispositif médical crânien implantable (100) selon l'une quelconque des revendications 1 à 9, dans lequel la portion bride supérieure (110) du boîtier (101) comprend un fond (114) annulaire généralement plat s'étendant latéralement par rapport à la portion inférieure (120).

11. Dispositif médical crânien implantable (100) selon l'une quelconque des revendications 1 à 10, comprenant un raccord de cathéter cérébral (130) s'étendant à partir de la portion inférieure (120), le raccord de cathéter cérébral comprenant une première lumière (132) en communication fluidique avec le premier trajet fluidique (192) et une deuxième lumière (134) en communication fluidique avec le deuxième trajet fluidique (194).

12. Système comprenant :
le dispositif médical crânien implantable (100) de la revendication 11 ; et
un cathéter cérébral (200) pouvant être accouplé fonctionnellement au raccord de cathéter cérébral (130),
dans lequel le cathéter cérébral comprend des première (212) et deuxième (214) lumières,
dans lequel la première lumière du cathéter cérébral est placée en communication fluidique avec la première lumière (132) du raccord de cathéter cérébral lorsque le cathéter cérébral est accouplé au raccord de cathéter cérébral, et
dans lequel la deuxième lumière du cathéter cérébral est placée en communication fluidique avec la deuxième lumière (134) du raccord de cathéter cérébral lorsque le cathéter cérébral est accouplé au raccord de cathéter cérébral.

13. Système comprenant :
le dispositif médical crânien implantable (100) de l'une quelconque des revendications 1 à 11 ou le système de la revendication 12 ; et
un dispositif de perfusion implantable (400) accouplé fonctionnellement au deuxième trajet fluidique (194).

14. Dispositif médical crânien implantable (100) selon la revendication 11, dans lequel le raccord de cathéter cérébral (130) comporte une fente (135) configurée pour recevoir une portion (215) d'un cathéter cérébral à lumière double (200) qui sépare une première lumière (212) du cathéter cérébral à lumière double d'une deuxième lumière (214) du cathéter cérébral à lumière double.

15. Système selon la revendication 12 ou la revendication 13, dans lequel le raccord de cathéter cérébral (130) comporte une fente (135) configurée pour recevoir une portion (215) du cathéter cérébral (200) qui sépare la première lumière (212) du cathéter cérébral de la deuxième lumière (214) du cathéter cérébral.
